# EUROPEAN PATENT SPECIFICATION

(11) **EP 2 178 535 B1**
(45) Date of publication and mention of the grant of the patent: **19.09.2012**
(21) Application number: 08827807.2
(22) Date of filing: 08.08.2008
(51) Int. Cl.: A61K 31/47, A61P 27/02

(54) **COMPOSITIONS AND METHODS FOR MODULATING ENDOPHTHALMITIS USING FLUOROQUINOLONES**
ZUSAMMENSETZUNGEN UND VERFAHREN ZUR MODULIERUNG VON ENDOPHTHALMITIS MITTELS FLUORCHINOLONEN
COMPOSITIONS ET PROCÉDÉS POUR MODULER UNE ENDOPHTALMITE EN UTILISANT DES FLUOROQUIONOLONES

(30) Priority: 21.08.2007 US 957005 P
(43) Date of publication of application: 28.04.2010
(73) Proprietor: Bausch & Lomb Incorporated, Rochester, NY 14604-2701 (US)
(72) Inventor: WARD, Keith, Wayne, Ontario, NY 14519 (US); ZHANG, Jinzhong, Pittsford, NY 14534 (US); JONASSE, Matthew, Scott, Webster, NY 14580 (US)
(74) Representative: Glas, Holger
(86) International application number: PCT/US2008/072552
(87) International publication number: WO 2009/026009

(56) References cited:
- EP-A- 0 493 608
- WO-A-02/100309
- WO-A-2008/091752
- WO-A-2008/115951
- BRUNNER ET AL: "P1680 Bactericidal activity of SS734, a novel fluoroquinolone, against pathogens associated with bacterial conjunctivitis" INTERNATIONAL JOURNAL OF ANTIMICROBIAL AGENTS, ELSEVIER SCIENCE, AMSTERDAM, NL, vol. 29, 1 March 2007 (2007-03-01), page S476, XP022038869 ISSN: 0924-8579
- BRUNNER ET AL: "P1679 In vitro activity of SS734, a novel fluoroquinolone, against pathogens associated with bacterial conjunctivitis" INTERNATIONAL JOURNAL OF ANTIMICROBIAL AGENTS, ELSEVIER SCIENCE, AMSTERDAM, NL, vol. 29, 1 March 2007 (2007-03-01), pages S475-S476, XP022038868 ISSN: 0924-8579
- CAMBAU ET AL: "P1665 Mode of action and resistance of the new fluoroquinolone BOL-303224-A" INTERNATIONAL JOURNAL OF ANTIMICROBIAL AGENTS, ELSEVIER SCIENCE, AMSTERDAM, NL, vol. 29, 1 March 2007 (2007-03-01), page S471, XP022038854 ISSN: 0924-8579

## Description

### BACKGROUND OF THE INVENTION

The present invention relates to compositions for use in a medicament for modulating endophthalmitis using fluoroquinolones.

The interface between the body and its environment is large, and thus presents many potential opportunities for invasion by environmental virulent pathogens. The outer tissues of the eye constitute parts of this interface, and thus, the eye and its surrounding tissues are also vulnerable to virulent microorganisms, the invasion and uncontrolled growth of which cause various types of ophthalmic infections, leading to inflammations, such as blepharitis, conjunctivitis, or keratitis, which can result in serious impairment of vision if untreated. The common types of microorganisms causing ophthalmic infections are viruses, bacteria, and fungi. These microorganisms may directly invade the surface of the eye, or permeate into the globe of the eye through trauma or surgery, or transmit into the eye through the blood stream or lymphatic system as a consequence of a systemic disease. The microorganisms may attack any part of the eye structure, including the conjunctiva, the cornea, the uvea, the vitreous body, the retina, and the optic nerve. Ocular or ophthalmic infections can cause severe pain, swollen and red tissues in or around the eye, and blurred and decreased vision.

The body's innate cascade is activated soon after invasion by a foreign pathogen begins. Leukocytes (neutrophils, eosinophils, basophils, monocytes, and macrophages) are attracted to the site of infection in an attempt to eliminate the foreign pathogen through phagocytosis. Leukocytes and some affected tissue cells are activated by the pathogens to synthesize and release proinflammatory cytokines such as IL-1β, IL-3, IL-5, IL-6, IL-8, TNF-α (tumor necrosis factor-α), GM-CSF (granulocyte-macrophage colony-stimulating factor), and MCP-1 (monocyte chemotactic protein-1). These released cytokines then further attract more immune cells to the infected site, amplifying the response of the immune system to defend the host against the foreign pathogen. For example, IL-8 and MCP-1 are potent chemoattractants for, and activators of, neutrophils and monocytes, respectively, while GM-CSF prolongs the survival of these cells and increases their response to other proinflammatory agonists. TNF-α can activate both types of cell and can stimulate further release of IL-8 and MCP-1 from them. IL-1 and TNF-α are potent chemoattractants for T and B lymphocytes, which are activated to produce antibodies against the foreign pathogen.

WO 2008//091752 describes a process of preparing quinolone carboxylic acid or its derivatives. In WO 2008/115951, reference is made to compositions comprising specific fluoroquinolones which can be used for treating, ameliorating or preventing infections caused by antibacterial drug-resistant bacteria. WO 02/100309 refers to the use of quinolone carboxylic acid formulations in the treatment of ocular and periocular infections.

Although an inflammatory response is essential to clear pathogens from the site of infection, a prolonged or overactive inflammatory response can be damaging to the surrounding tissues. For example, inflammation causes the blood vessels at the infected site to dilate to increase blood flow to the site. As a result, these dilated vessels become leaky. After prolonged inflammation, the leaky vessels can produce serious edema in, and impair the proper functioning of, the surrounding tissues (see; e.g., V.W.M. van Hinsbergh, Arteriosclerosis, Thrombosis, and Vascular Biology, Vol. 17, 1018 (1997)). In addition, a continued dominating presence of macrophages at the injured site continues the production of toxins (such as reactive oxygen species) and matrix-degrading enzymes (such as matrix metalloproteinases) by these cells, which are injurious to both the pathogen and the host's tissues. Therefore, a prolonged or overactive inflammation should be controlled to limit the unintended damages to the body and to hasten the body's recovery process.

Endophthalmitis is an inflammation of the intraocular cavities (i.e., the anterior and posterior chambers of the eye) and surrounding tissues. In most cases, an infection, which can be caused by bacteria, fungi, viruses, or parasites, triggers this inflammation. Post-operative endophthalmitis is the most common species of endophthalmitis and results from bacterial infection after cataract, glaucoma, or retinal surgery, or radial keratotomy. The most common bacteria associated with endophthalmitis is *Staphylococcus epidermidis.* Other *Staphylococus, Streptococcus,* and *Pseudomonas* species also have been found in endophthalmitis cases. Non-infectious endophthalmitis can be a result of penetrating injuries to the eye or of retained native materials after cataract surgery. Hematogenous endophthalmitis is caused by an infection spreading through the bloodstream and settling in the eye. Without prompt treatment, endophthalmitis can cause loss of vision.

Glucocorticoids (also referred to herein as "corticosteroids") represent one of the most effective clinical treatment for a range of inflammatory conditions, including acute inflammation. However, steroidal drugs can have side effects that threaten the overall health of the patient.

It is known that certain glucocorticoids have a greater potential for elevating intraocular pressure ("IOP") than other compounds in this class. For example, it is known that prednisolone, which is a very potent ocular anti-inflammatory agent, has a greater tendency to elevate IOP than fluorometholone, which has moderate ocular anti-inflammatory activity. It is also known that the risk of IOP elevations associated with the topical ophthalmic use of glucocorticoids increases over time. In other words, the chronic (i.e., long-term) use of these agents increases the risk of significant IOP elevations. Unlike acute ocular inflammation associated with physical trauma or infection of the outer surface of the anterior portion of the eye, which requires short-term therapy on the order of a few weeks, infection and inflammation of the posterior portion of the eye can require treatment for extended periods of time, generally several months or more. This chronic use of corticosteroids significantly increases the risk of IOP elevations. In addition, use of corticosteroids is also known to increase the risk of cataract formation in a dose- and duration-dependent manner. Once cataracts develop, they may progress despite discontinuation of corticosteroid therapy.

Chronic administration of glucocorticoids also can lead to drug-induced osteoporosis by suppressing intestinal calcium absorption and inhibiting bone formation. Other adverse side effects of chronic administration of glucocorticoids include hypertension, hyperglycemia, hyperlipidemia (increased levels of triglycerides) and hypercholesterolemia (increased levels of cholesterol) because of the effects of these drugs on the body metabolic processes.

Therefore, there is a continued need to provide improved pharmaceutical compounds, compositions, and methods for modulating endophthamitis.

### SUMMARY OF THE INVENTION

In general, the present invention provides compositions for use in a medicament for modulating inflammatory endophthalmitis using fluoroquinolones. More precisely, the present invention refers to a composition as defined in claim 1.

In another aspect, said endophthalmitis is selected from the group consisting of post-operative endophthalmitis, post-traumatic endophthalmitis, non-infectious endophthalmitis, panophthalmitis, hematogenous endophthalmitis, and combinations thereof. Panophthalmitis is inflammation of all coats of the eye, including the intraocular structures.

In another aspect, the present invention provides compositions comprising a fluoroquinolone having Formula 1 or a salt thereof for use in a medicament for modulating inflammatory endophthalmitis 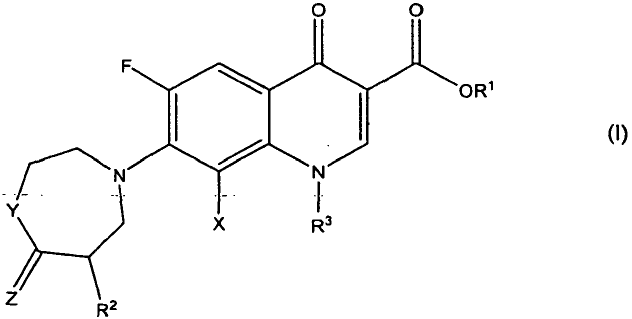 wherein R¹ is selected from the group consisting of hydrogen, unsubstituted lower alkyl groups, substituted lower alkyl groups, cycloalkyl groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, and groups that can be hydrolyzed in living bodies; R² is selected from the group consisting of hydrogen, unsubstituted amino group, and amino groups substituted with one or two lower alkyl groups; R³ is selected from the group consisting of hydrogen,
unsubstituted lower alkyl groups, substituted lower alkyl groups, cycloalkyl groups, unsubstituted lower alkoxy groups, substituted lower alkoxy groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, unsubstituted C₅-C₂₄ aryloxy groups, substituted C₅-C₂₄ aryloxy groups, unsubstituted C₅-C₂₄ heteroaryloxy groups, substituted C₅-C₂₄ heteroaryloxy groups, and groups that can be hydrolyzed in living bodies; X is selected from the group consisting of halogen atoms; Y is selected from the group consisting of CH₂, O, S, SO, SO₂, and NR⁴, wherein R⁴ is selected from the group consisting of hydrogen, unsubstituted lower alkyl groups, substituted lower alkyl groups, and cycloalkyl groups; and Z is selected from the group consisting of oxygen and two hydrogen atoms.

In yet another aspect, such endophthalmitis results from an infection caused by bacteria, viruses, fungi, or protozoans.

In still another aspect, such endophthamitis results from a physical injury or trauma to the eye.

Other features and advantages of the present invention will become apparent from the following detailed description and claims and the appended figures.

### BRIEF DESCRIPTION OF THE FIGURES

Figure 1 shows the effect of moxifloxacin and compound having Formula IV ("BOL-303224-A") on LPS-simulated GM-CSF, IL-1β, and IL-8, IP-10, MCP-1, and MIP-1α production in THP-1 monocytes.
Figure 2 shows the effect of moxifloxacin and compound having Formula IV on LPS-stimulated G-CSF, IL-1α, IL-1ra, IL-6, and VEGF production in THP- 1 monocytes.
Figure 3 shows the effect of moxifloxacin and compound having Formula IV on LPS-simulated IL-12p40 production in THP-1 monocytes.

### DETAILED DESCRIPTION OF THE INVENTION

As used herein, the term "control" includes reduction, amelioration, alleviation, and prevention.

As used herein, the term "lower alkyl" or "lower alkyl group" means a C₁-C₁₅ linear- or branched-chain saturated aliphatic hydrocarbon monovalent group, which may be unsubstituted or substituted. The group may be partially or completely substituted with halogen atoms (F, Cl, Br, or I). Non-limiting examples of lower alkyl groups include methyl, ethyl, n-propyl, 1-methylethyl (isopropyl), n-butyl, n-pentyl, 1,1-dimethylethyl (t-butyl), and the like. It may be abbreviated as "Alk". Preferably, a lower alkyl group comprises 1-10 carbon atoms. More preferably, a lower alkyl group comprises 1-5 carbon atoms.

As used herein, the term "lower alkoxy" or "lower alkoxy group" means a C₁-C₁₅ linear- or branched-chain saturated aliphatic alkoxy monovalent group, which may be unsubstituted or substituted. The group may be partially or completely substituted with halogen atoms (F, Cl, Br, or I). Non-limiting examples of lower alkoxy groups include methoxy, ethoxy, n-propoxy, 1-methylethoxy (isopropoxy), n-butoxy, n-pentoxy, t-butoxy, and the like. Preferably, a lower alkyloxy group comprises 1-10 carbon atoms. More preferably, a lower alkyloxy group comprises 1-5 carbon atoms.

The term "cycloalkyl" or "cycloalkyl group" means a stable aliphatic saturated 3-to 15-membered monocyclic or polycyclic monovalent radical consisting solely of carbon and hydrogen atoms which may comprise one or more fused or bridged ring(s), preferably a 3- to 7-membered monocyclic rings. Other exemplary embodiments of cycloalkyl groups include 7- to 10-membered bicyclic rings. Unless otherwise specified, the cycloalkyl ring may be attached at any carbon atom which results in a stable structure and, if substituted, may be substituted at any suitable carbon atom which results in a stable structure. Exemplary cycloalkyl groups include cyclopropyl, cyclobutyl, cyclopentyl, cyclohexyl, cycloheptyl, cyclooctyl, cyclononyl, cyclodecyl, norbornyl, adamantyl, tetrahydronaphthyl (tetralin), 1-decalinyl, bicyclo[2.2.2]octanyl, 1-methylcyclopropyl, 2-methylcyclopentyl, 2-methylcyclooctyl, and the like.

As used herein, the term "aryl" or "aryl group" means an aromatic carbocyclic monovalent or divalent radical. In some embodiments, the aryl group has a number of carbon atoms from 5 to 24 and has a single ring (e.g., phenyl or phenylene), multiple condensed rings (e.g., naphthyl or anthranyl), or multiple bridged rings (e.g., biphenyl). Unless otherwise specified, the aryl ring may be attached at any suitable carbon atom which results in a stable structure and, if substituted, may be substituted at any suitable carbon atom which results in a stable structure. Non-limiting examples of aryl groups include phenyl, naphthyl, anthryl, phenanthryl, indanyl, indenyl, biphenyl, and the like. It may be abbreviated as "Ar". Preferably, an aryl group comprises 5-14 carbon atoms. More preferably, an aryl group comprises 5-10 carbon atoms.

The term "heteroaryl" or "heteroaryl group" means a stable aromatic monocyclic or polycyclic monovalent or divalent radical, which may comprise one or more fused or bridged ring(s). In some embodiments, the heteroaryl group has 5-24 members, preferably a 5- to 7-membered monocyclic or 7- to 10-membered bicyclic radical. The heteroaryl group can have from one to four heteroatoms in the ring(s) independently selected from nitrogen, oxygen, and sulfur, wherein any sulfur heteroatoms may optionally be oxidized and any nitrogen heteroatom may optionally be oxidized or be quaternized. Unless otherwise specified, the heteroaryl ring may be attached at any suitable heteroatom or carbon atom which results in a stable structure and, if substituted, may be substituted at any suitable heteroatom or carbon atom which results in a stable structure. Non-limiting examples of heteroaryls include furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, triazinyl, indolizinyl, azaindolizinyl, indolyl, azaindolyl, diazaindolyl, dihydroindolyl, dihydroazaindoyl, isoindolyl, azaisoindolyl, benzofuranyl, furanopyridinyl, furanopyrimidinyl, furanopyrazinyl, furanopyridazinyl, dihydrobenzofuranyl, dihydrofuranopyridinyl, dihydrofuranopyrimidinyl, benzothienyl, thienopyridinyl, thienopyrimidinyl, thienopyrazinyl, thienopyridazinyl, dihydrobenzothienyl, dihydrothienopyridinyl, dihydrothienopyrimidinyl, indazolyl, azaindazolyl, diazaindazolyl, benzimidazolyl, imidazopyridinyl, benzthiazolyl, thiazolopyridinyl, thiazolopyrimidinyl, benzoxazolyl, benzoxazinyl, benzoxazinonyl, oxazolopyridinyl, oxazolopyrimidinyl, benzisoxazolyl, purinyl, chromanyl, azachromanyl, quinolizinyl, quinolinyl, dihydroquinolinyl, tetrahydroquinolinyl, isoquinolinyl, dihydroisoquinolinyl, tetrahydroisoquinolinyl, cinnolinyl, azacinnolinyl, phthalazinyl, azaphthalazinyl, quinazolinyl, azaquinazolinyl, quinoxalinyl, azaquinoxalinyl, naphthyridinyl, dihydronaphthyridinyl, tetrahydronaphthyridinyl, pteridinyl, carbazolyl, acridinyl, phenazinyl, phenothiazinyl, and phenoxazinyl, and the like.

Glucocorticoids ("GCs") are among the most potent drugs used for the treatment of allergic and chronic inflammatory diseases or of inflammation resulting from infections. However, as mentioned above, long-term treatment with GCs is often associated with numerous adverse side effects, such as diabetes, osteoporosis, hypertension, glaucoma, or cataract. These side effects, like other physiological manifestations, are results of aberrant expression of genes responsible for such diseases. Research in the last decade has provided important insights into the molecular basis of GC-mediated actions on the expression of GC-responsive genes. GCs exert most of their genomic effects by binding to the cytoplasmic GC receptor ("GR"). The binding of GC to GR induces the translocation of the GC-GR complex to the cell nucleus where it modulates gene transcription either by a positive (transactivation) or negative (transrepression) mode of regulation. There has been growing evidence that both beneficial and undesirable effects of GC treatment are the results of undifferentiated levels of expression of these two mechanisms; in other words, they proceed at similar levels of effectiveness. Although it has not yet been possible to ascertain the most critical aspects of action of GCs in chronic inflammatory diseases, there has been evidence that it is likely that the inhibitory effects of GCs on cytokine synthesis are of particular importance. GCs inhibit the transcription, through the transrepression mechanism, of several cytokines that are relevant in inflammatory diseases, including IL-1β (interleukin-1β), IL-2, IL-3, IL-6, IL-11, TNF-α (tumor necrosis factor-α), GM-CSF (granulocyte-macrophage colony-stimulating factor), and chemokines that attract inflammatory cells to the site of inflammation, including IL-8, RANTES, MCP-1 (monocyte chemotactic protein-1), MCP-3, MCP-4, MIP-1α (macrophage-inflammatory protein-1α), and eotaxin. P.J. Bames, Clin. Sci., Vol. 94, 557-572 (1998). On the other hand, there is persuasive evidence that the synthesis of IκB kinases, which are proteins having inhibitory effects on the NF-κB proinflammatory transcription factors, is increased by GCs. These proinflammatory transcription factors regulate the expression of genes that code for many inflammatory proteins, such as cytokines, inflammatory enzymes, adhesion molecules, and inflammatory receptors. S. Wissink et al., Mol. Endocrinol., Vol. 12, No. 3, 354-363 (1998); P.J. Barnes and M. Karin, New Engl. J. Med., Vol. 336, 1066-1077 (1997). Thus, both the transrepression and transactivation functions of GCs directed to different genes produce the beneficial effect of inflammatory inhibition. On the other hand, steroid-induced diabetes and glaucoma appear to be produced by the transactivation action of GCs on genes responsible for these diseases. H. Schäcke et al., Pharmacol. Ther., Vol. 96, 23-43 (2002). Thus, while the transactivation of certain genes by GCs produces beneficial effects, the transactivation of other genes by the same GCs can produce undesired side effects. Therefore, it is very desirable to provide pharmaceutical compounds, compositions, and methods for modulating inflammation without the undesired side effects of GC therapy.

In general, the present invention provides compositions for use in a medicament for modulating endophthalmitis using fluoroquinolones.

In another aspect, such endophthamitis is selected from the group consisting of post-operative endophthalmitis, post-traumatic endophthalmitis, non-infectious endophthalmitis, panophthalmitis, hematogenous endophthalmitis, and combinations thereof.

In another aspect, the present invention provides compositions comprising a fluoroquinolone having Formula 1 or a salt thereof for use in a medicament for modulating inflamatory endophthalmitis 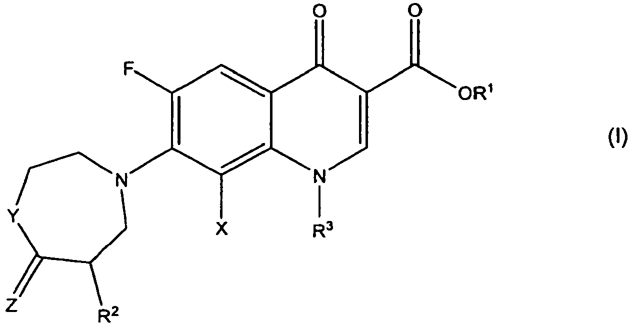 wherein R¹ is selected from the group consisting of hydrogen, unsubstituted lower alkyl groups, substituted lower alkyl groups, cycloalkyl groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, and groups that can be hydrolyzed in living bodies; R² is selected from the group consisting of hydrogen, unsubstituted amino group, and amino groups substituted with one or two lower alkyl groups; R³ is selected from the group consisting of hydrogen, unsubstituted lower alkyl groups, substituted lower alkyl groups, cycloalkyl groups, unsubstituted lower alkoxy groups, substituted lower alkoxy groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, unsubstituted C₅-C₂₄ aryloxy groups, substituted C₅-C₂₄ aryloxy groups, unsubstituted C₅-C₂₄ heteroaryloxy groups, substituted C₅-C₂₄ heteroaryloxy groups, and groups that can be hydrolyzed in living bodies; X is selected from the group consisting of halogen atoms; Y is selected from the group consisting of CH₂, O, S, SO, SO₂, and NR⁴, wherein R⁴ is selected from the group consisting of hydrogen, unsubstituted lower alkyl groups, substituted lower alkyl groups, and cycloalkyl groups; and Z is selected from the group consisting of oxygen and two hydrogen atoms.

In still another aspect, a composition of the present invention for use in a medicament for modulating endophthalmitis comprises a member of a family of fluoroquinolones having Formula II or salts thereof, 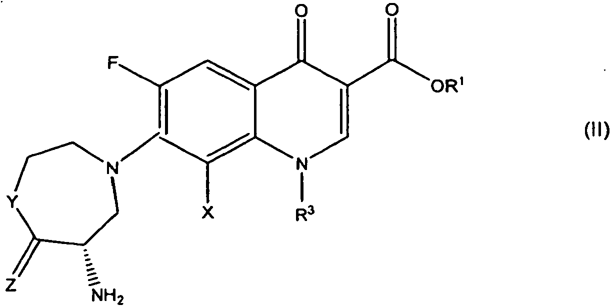 wherein R¹, R³, X, Y, and Z have the meanings as disclosed above;

In one aspect, R¹ is selected from the group consisting of hydrogen, C₁-C₅ (or alternatively, C₁-C₃) substituted and unsubstituted alkyl groups, C₃-C₁₀ (or alternatively, C₃-C₅) cycloalkyl groups, C₅-C₁₄ (or alternatively, C₆-C₁₄, or C₅-C₁₀, or C₆-C₁₀) substituted and unsubstituted aryl groups, C₅-C₁₄ (or alternatively, C₆-C₁₄, or C₅-C₁₀, or C₆-C₁₀) substituted and unsubstituted heteroaryl groups, and groups that can be hydrolyzed in living bodies. In one embodiment, R¹ is selected from the group consisting of C₁-C₅ (or alternatively, C₁-C₃) substituted and unsubstituted alkyl groups.

In another aspect, R² is selected from the group consisting of unsubstituted amino group and amino groups substituted with one or two C₁-C₅ (or alternatively, C₁-C₃) alkyl groups.

In still another aspect, R³ is selected from the group consisting of hydrogen, C₁-C₅ (or alternatively, C₁-C₃) substituted and unsubstituted alkyl groups, C₃-C₁₀ (or alternatively, C₃-C₅) cycloalkyl groups, C₁-C₅ (or alternatively, C₁-C₃) substituted and unsubstituted alkoxy groups, C₅-C₁₄ (or alternatively, C₆-C₁₄, or C₅-C₁₀, or C₆-C₁₀) substituted and unsubstituted aryl groups, C₅-C₁₄ (or alternatively, C₆-C₁₄, or C₅-C₁₀, or C₆-C₁₀) substituted and unsubstituted heteroaryl groups, and C₅-C₁₄ (or alternatively, C₆-C₁₄, or C₅-C₁₀, or C₆-C₁₀) substituted and unsubstituted aryloxy groups. In one embodiment, R³ is selected from the group consisting of C₃-C₁₀ (or alternatively, C₃-C₅) cycloalkyl groups.

In yet another aspect, X is selected from the group consisting of Cl, F, and Br. In one embodiment, X is Cl. In another embodiment, X is F.

In a further aspect, Y is CH₂. In still another aspect, Z comprises two hydrogen atoms.

In still another aspect, Y is NH, Z is O, and X is Cl.

In another aspect, a composition of the present invention further comprises a pharmaceutically acceptable carrier.

Some non-limiting members of the family of compounds having Formula I are shown in Table 1. Other compounds of the family not listed in Table I are also suitable in selected situations.

**Table 1**

| Some Selected Fluoroquinolones | | | | | | |
|---|---|---|---|---|---|---|
| Compound | R¹ | R² | R³ | X | Y | Z |
| I | H | H | CH₃ | Cl | CH₂ | 2 H |
| 2 | H | NH₂ | CH₃ | Cl | CH₂ | 2 H |
| 3 | H | NH₂ | cyclopropyl | Cl | CH₂ | 2 H |
| 4 | H | NH(CH₃) | cyclopropyl | Cl | CH₂ | 2 H |
| 5 | H | N(CH₃)₂ | cyclopropyl | Cl | CH₂ | 2 H |
| 6 | CH₃ | NH₂ | cyclopropyl | Cl | CH₂ | 2 H |
| 7 | C₂H₅ | NH₂ | cyclopropyl | Cl | CH₂ | 2 H |
| 8 | H | NH₂ | cyclopropyl | F | CH₂ | 2 H |
| 9 | H | NH₂ | cyclopropyl | Br | CH₂ | 2 H |
| 10 | H | NH(C₂H₅) | cyclopropyl | Cl | CH₂ | 2 H |
| 11 | H | NH(C₃H₇) | cyclopropyl | F | CH₂ | 2 H |
| 12 | H | NH₂ | cyclopentyl | Cl | CH₂ | 2 H |
| 13 | H | NH₂ | cyclopropyl | Cl | CH₂ | O |
| 14 | H | NH₂ | cyclopropyl | F | CH₂ | O |
| 15 | H | NH₂ | cyclopropyl | Br | CH₂ | O |
| 16 | H | NH₂ | cyclopropyl | Cl | CH(C₂H₅) | O |
| 17 | CH₃ | NH₂ | cyclopropyl | Cl | CH₂ | O |
| 18 | CH₃ | NH(CH₃) | cyclopropyl | Cl | CH₂ | O |
| 19 | CH₃ | N(CH₃)₂ | cyclopropyl | Cl | CH₂ | O |
| 20 | CH₃ | NH(C₃H₇) | cyclopropyl | Cl | CH₂ | O |
| 21 | CH₃ | NH(C₂H₅) | cyclopropyl | Cl | CH₂ | O |
| 22 | CH₃ | N(CH₃)(C₂H₅) | cyclopropyl | Cl | CH₂ | O |
| 23 | H | NH₂ | cyclopropyl | Cl | NH | O |
| 24 | CH₃ | NH(CH₃) | cyclopropyl | Cl | NH | O |
| 25 | H | 2H | cyclopropyl | Cl | NH | O |

In one embodiment, the fluoroquinolone carboxylic acid included in a composition and used in a method of the present invention has Formula III.

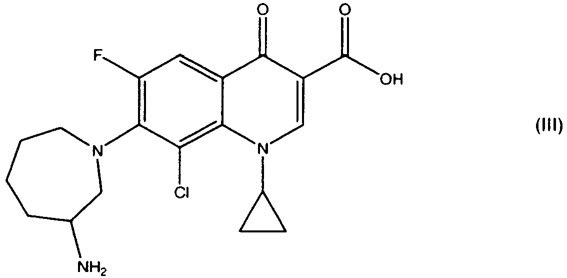

In another embodiment, the fluoroquinolone carboxylic acid included in a composition and used in a method of the present invention has Formula IV, V, or VI.

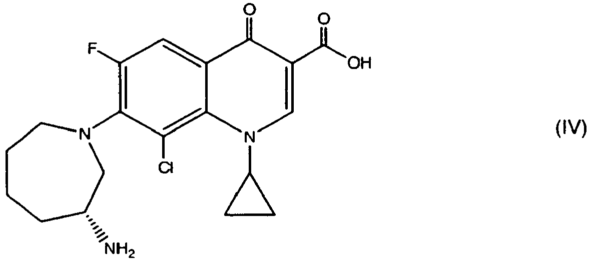

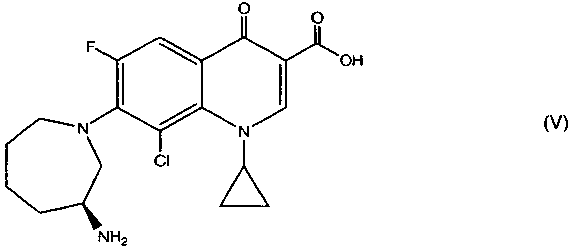

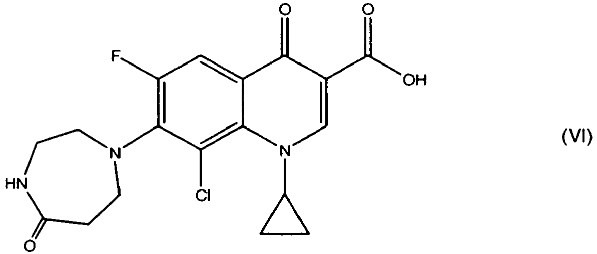

In still other embodiments, the fluoroquinolone carboxylic acid included in a composition and used in a method of the present invention has Formula VII or VIII.

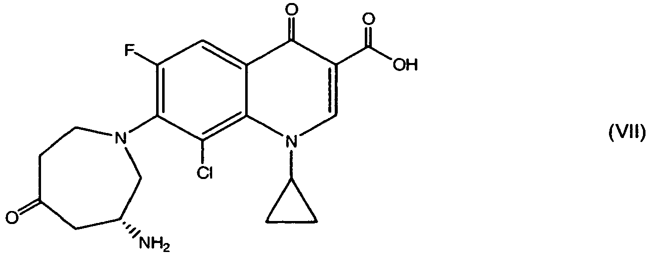

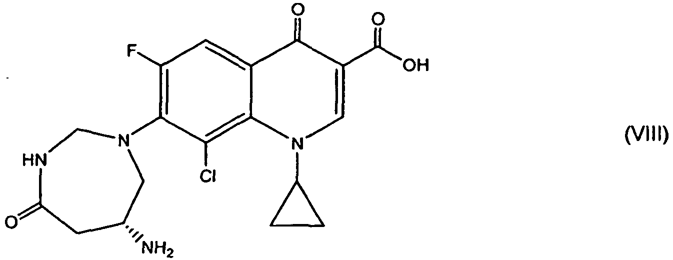

In still another aspect, a composition of the present invention comprises an enantiomer of one of the compounds having Formula I, II, or III,

In still another aspect, a composition of the present invention comprises a mixture of enantiomers of one of the compounds having Formula I, II, or III .

A fluoroquinolone disclosed herein can be produced by a method disclosed in U.S. Patents 5,447,926 and 5,385,900.

In yet another aspect, such an infection is caused by bacteria, viruses, fungi, protozoans, or combinations thereof.

In yet another aspect, the present invention provides a composition for use in a medicament for modulating an inflammatory response accompanying an ocular surgery, wherein such a composition comprises one of the fluoroquinolones having Formula I, II, III, IV, V, VI, VII, or VIII, and such a method employs such a composition. Non-limiting examples of such ocular surgery include cataract surgery, glaucoma surgery, retinal surgery, and radial keratotomy.

In yet another aspect, the present invention provides composition for use in a medicament for treating or controlling endophthalmitis in a subject, which compositions cause a lower level of at least an adverse side effect than compositions comprising at least a prior-art glucocorticoid used to treat or control said endophthalmitis.

In one aspect, a level of said at least an adverse side effect is determined in vivo or in vitro. For example, a level of said at least an adverse side effect is determined in vitro by performing a cell culture and determining the level of a biomarker associated with said side effect. Such biomarkers can include proteins (e.g., enzymes), lipids, sugars, and derivatives thereof that participate in, or are the products of, the biochemical cascade resulting in the adverse side effect. Representative in vitro testing methods are further disclosed hereinbelow.

In still another aspect, said at least an adverse side effect is selected from the group consisting of glaucoma, cataract, hypertension, hyperglycemia, hyperlipidemia (increased levels of triglycerides), and hypercholesterolemia (increased levels of cholesterol).

In another embodiment, a level of said at least an adverse side effect is determined at about one day after said composition is first administered to, and are present in, said subject. In another embodiment, a level of said at least an adverse side effect is determined about 14 days after said composition is first administered to, and are present in, said subject. In still another embodiment, a level of said at least an adverse side effect is determined about 30 days after said composition is first administered to, and are present in, said subject. Alternatively, a level of said at least an adverse side effect is determined about 2. 3, 4, 5, or 6 months after said compounds or compositions are first administered to, and are present in, said subject.

In another aspect, said at least a prior-art glucocorticoid used to treat, control, reduce, or ameliorate the same conditions is administered to said subject at a dose and a frequency sufficient to produce an equivalent beneficial effect on said condition to a composition of the present invention after about the same elapsed time.

In still another aspect, said at least a prior-art glucocorticoid is selected from the group consisting of 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, their physiologically acceptable salts, combinations thereof, and mixtures thereof. In one embodiment, said at least a prior-art glucocorticoid is selected from the group consisting of dexamethasone, prednisone, prednisolone, methylprednisolone, medrysone, triamcinolone, loteprednol etabonate, physiologically acceptable salts thereof, combinations thereof, and mixtures thereof. In another embodiment, said at least a prior-art glucocorticoid is acceptable for ophthalmic uses.

### TESTING 1: Inhibition of LPS-Induced Cytokine Expression in Human THP-1 Monocytes by Compound Having Formula IV and Moxifloxacin

### Experimental Method

Human THP-1 monocytes (ATCC TIB 202) were purchased from American Type Culture Collection (Manassas, Virginia) and maintained in RPMI 1640 medium (Invitrogen, Carlsbad, California) supplemented with 10% fetal bovine serum ("FBS", Invitrogen, Carlsbad, California), 100 U/mL of penicillin (Invitrogen, Carlsbad, California), and 100 µg/mL of streptomycin (Invitrogen, Carlsbad, California) at 37°C in a humidified incubator with 5% CO₂. THP-1 cells were pre-cultured in RPMI 1640 medium containing 10% dialyzed serum for 24 h. Cells were seeded in 24-well plates in RPMI 1640 medium containing 2% dialyzed serum (purchased from Hyclone, Loga, Utah) and treated with vehicle (DMSO, dimethyl sulfoxide), 10 µg/mL LPS (Sigma Aldrich, St. Louis, Missouri), 0.1, 1, 10 or 30 µg/mL moxifloxacin (Neuland Laboratories, Hyderabad, India), 0.1. 1, 10 or 30 µg/mL compound having Formula IV (also referred to herein as "BOL-303224-A," Bausch & Lomb Incorporated, Rochester, New York), 10 µg/ml LPS + 0.1, 1, 10 or 30 µg/mL moxifloxacin, or 10 µg/ml LPS + 0.1, 1, 10 or 30 µg/mL compound having Formula IV for 18 hours. Each treatment was performed in triplicate.

### Multiplex Luminex

Samples were analyzed using multiplex bead technology, which utilizes microspheres as the solid support for immunoassays and allows the analysis of all cytokines from each sample (D.A. Vignali, J. Immunol. Methods, Vol. 243, 243-255 (2000)). Sixteen cytokines were measured according to the manufacturer's instructions. Briefly, 50 µL of medium samples were incubated with antibody-coated capture beads overnight at 4°C. Washed beads were further incubated with biotin-labelled anti-human cytokine antibodies for 2 h at room temperature followed by incubation with streptavidin-phycoerythrin for 30 min. Samples were analyzed using Luminex 200™ (Luminex, Austin, Texas) and Beadview software v1.0 (Upstate Cell Signaling Solutions, Temecula, California). Standard curves of known concentrations of recombinant human cytokines were used to convert fluorescence units (median fluorescence intensity) to cytokine concentration in pg/mL. Only the linear portions of the standard cuves were used to quantify cytokine concentrations, and in instances where the fluorescence reading exceeded the linear range of the standard curve, an appropriate dilution was performed to ensure that the concentration was in the linear portion of the curve.

### Cellular Metabolic Function

Cellular metabolic competence was determined by an AlamarBlue assay (J. O'Brien et al., FEBS J., Vol. 267, 5421-5426 (2000)). Briefly, after removal of medium, cells were incubated with 1:10 diluted AlamarBlue solution (Biosource, Camarillo, California) for 3 hours at 37°C in a humidified incubator with 5% CO₂. The plate was read fluorometrically by excitation at 530-560 nm and emission at 590 nm. Relative fluorescence units ("RFU") were used to determine cell viability

### Data Analysis and Statistics

All cytokine concentrations (pg/mL) were expressed as mean ± standard deviation. Statistical analysis comparing effects of treatment across groups was performed using a one-way ANOVA with a Dunnett's post-hoc comparison test using either vehicle control or LPS treatment as references. For all assays, *p* ≤ 0.05 was predetermined as the criterion of statistical significance.

### Results

In no instance did any of the treatments produce a statistically significant effect on cellular metabolic activity as measured by the AlamarBlue assay (data not shown). The overall results from the studies determining cytokine levels in the culture medium from these various treatment groups are summarized in Table 2. Substantial levels of 14 out of the 16 cytokines in the assay were detectable in culture media from THP-1 monocytes, with all cytokines except EGF and IL-7 affected. Exposure of THP-1 monocytes to 10 µg/mL of LPS for 18 hours resulted in a significant increase of 13 out of the 14 detectable cytokines; the amount of VEGF in THP-1 monocyte culture medium also increased, but the increase did not attain statistical significance.

**Table 2**

| Summary of inhibition of LPS-stimulated cytokine production by moxifloxacin and Compound Having Formula IV in human THP-1 monocytes | | | | | | | | |
|---|---|---|---|---|---|---|---|---|
| Cytokine | Inhibited by Moxifloxacin at µg/mL | | | | Inhibited by Compound Having Formula IV at µg/mL | | | |
| | 0.1 | 1 | 10 | 30 | 0.1 | 1 | 10 | 30 |
| Fractalkine | | | | | | | | |
| G-CSF | | | | X | | X | X | X |
| GM-CSF | | | | X | | | | X |
| IL-12p40 | | X | X | X | | | | X |
| IL-1α | | | | X | X | X | X | X |
| IL-1β | | | | X | | | | X |
| IL-1ra | | | X | X | | X | X | X |
| IL-6 | | | X | | | X | X | X |
| IL-8 | | | | X | | | | X |
| IP-10 | | | | X | | | | X |
| MCP-1 | | | | | | | | X |
| MIP-1α | | | | X | | | | X |
| RANTES | | | | | | | | |
| VEGF | | | | X | | | X | X |

| | | | | | | | | |
|---|---|---|---|---|---|---|---|---|
| Note: "X" signifies significant inhibition at a particular concentration. | | | | | | | | |

Both moxifloxacin and compound having Formula IV significantly inhibited LPS-induced cytokine production in THP-1 monocytes. For moxifloxacin, a significant inhibitory effect was observed at 1 µg/ml for IL-12p40, at 10 µg/ml for IL-1ra and IL-6. and at 30 µg/ml for G-CSF, GM-CSF, IL-1α, IL-1β, IL-8, IP-10, and MIP-1α (Table 1). For compound having Formula IV, a significant inhibitory effect was observed at 0.1 µg/ml for IL-1α, at 1 µg/ml for G-CSF, IL-1ra and IL-6, and at 30 µg/ml for GM-CSF, IL-12p40, IL-1β, IL-Ira, IL-8, IP-10, MCP-1 and MIP-1 a (Table 2). Neither moxifloxacin nor compound having Formula IV altered LPS-stimulated production of RANTES or fractalkine.

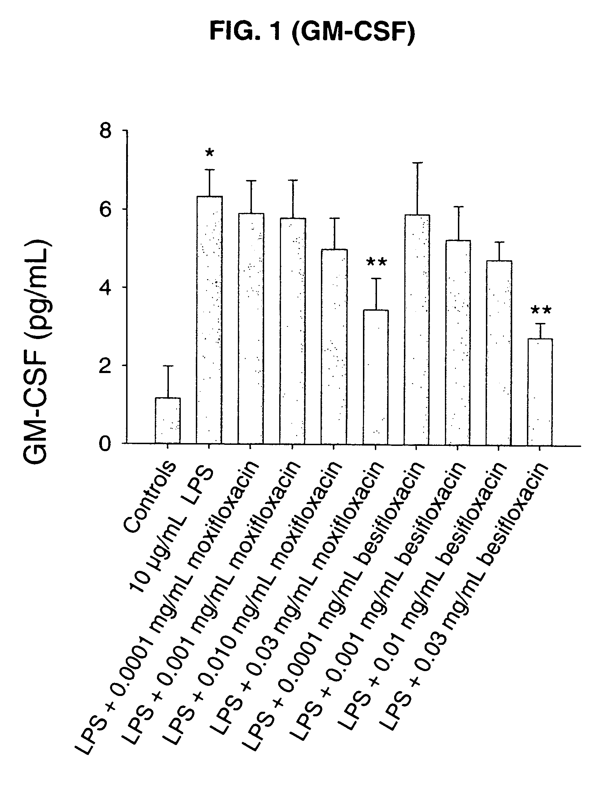

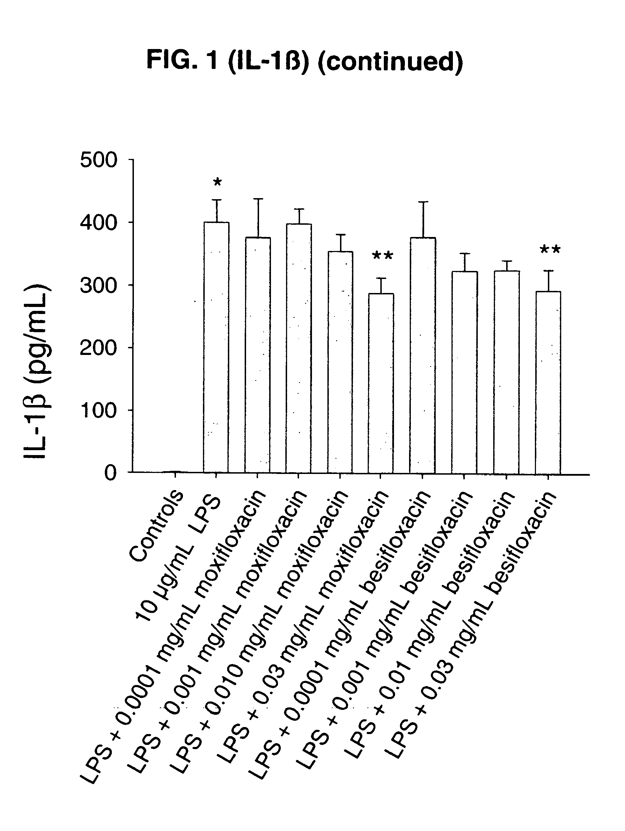

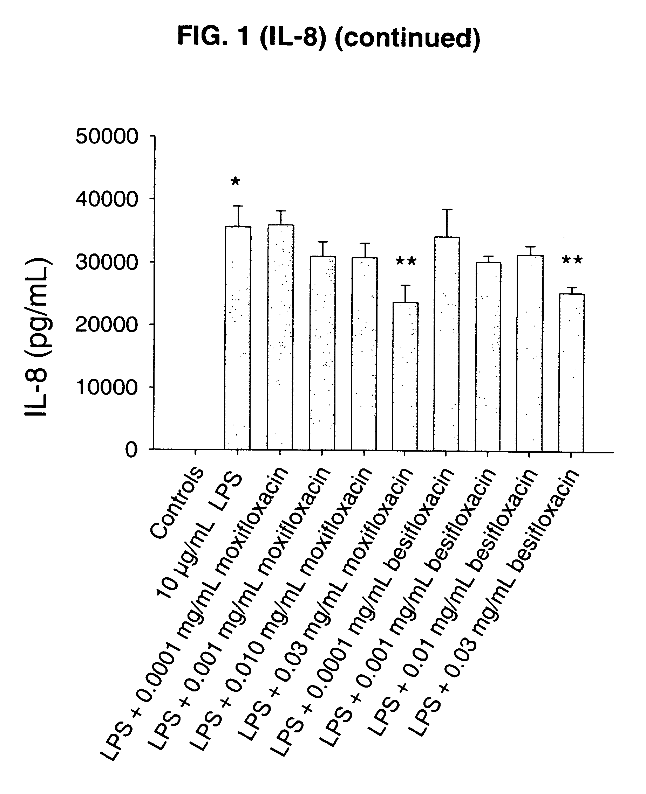

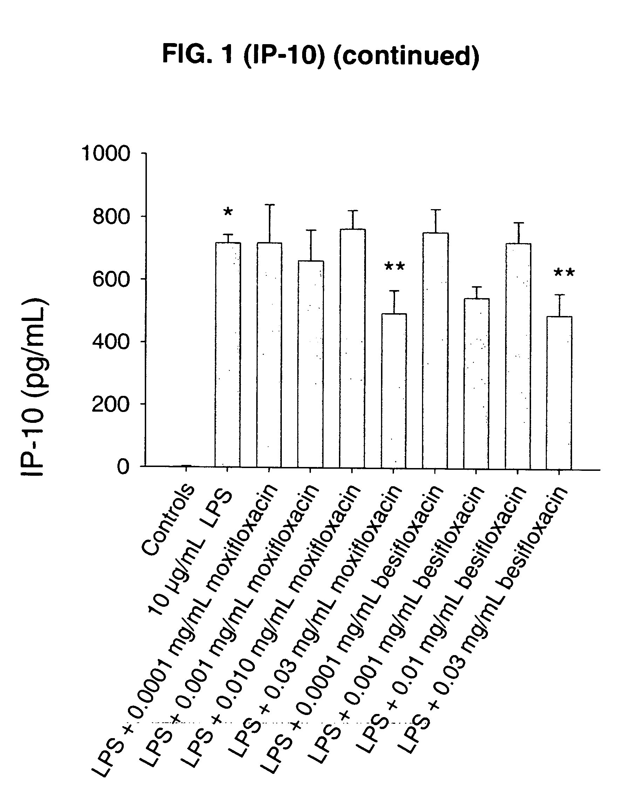

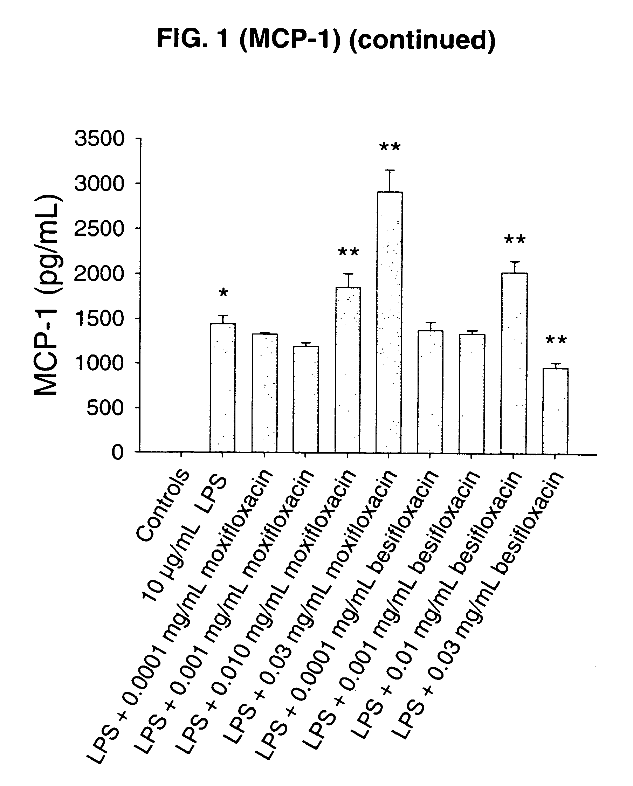

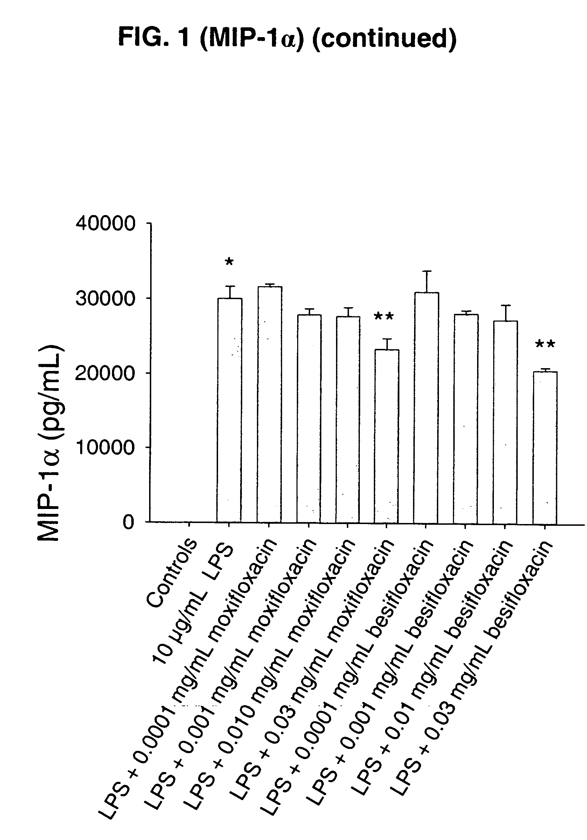

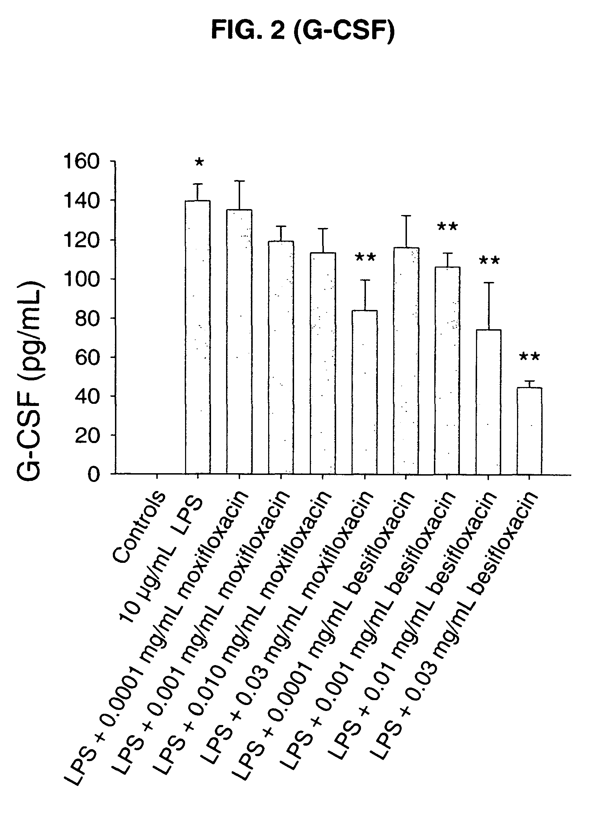

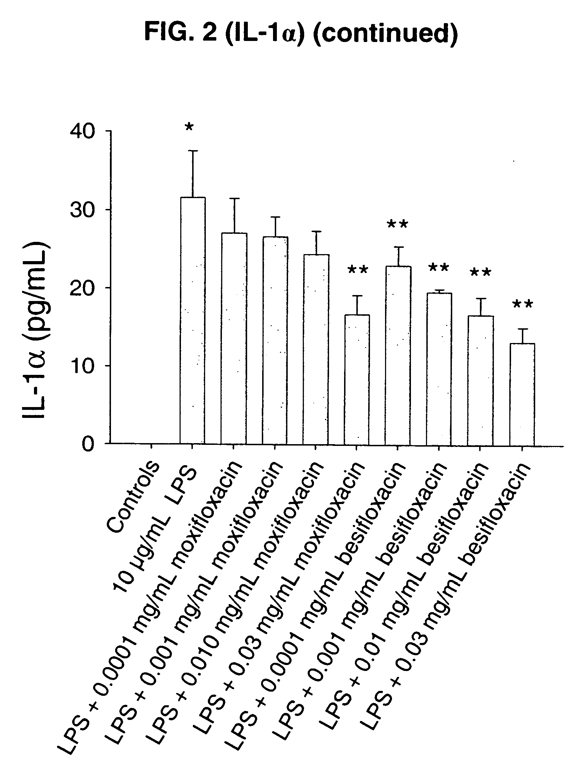

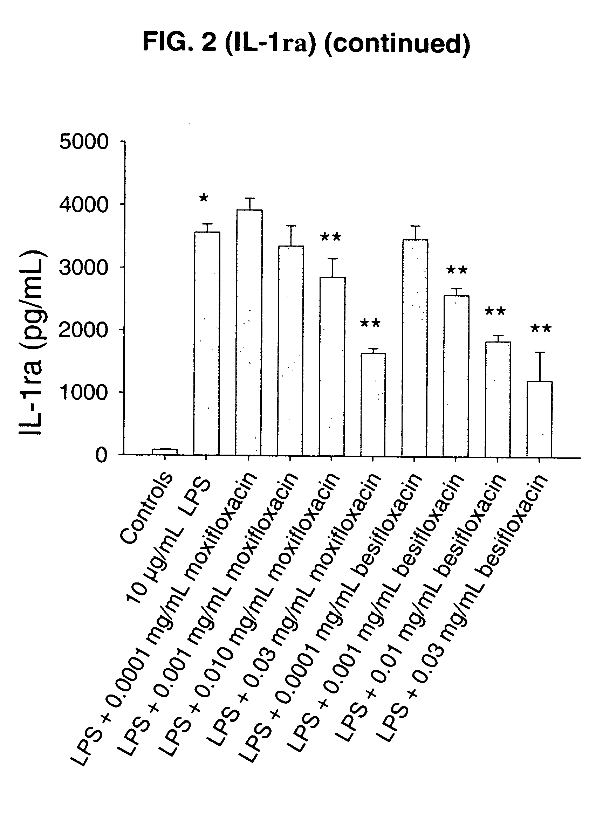

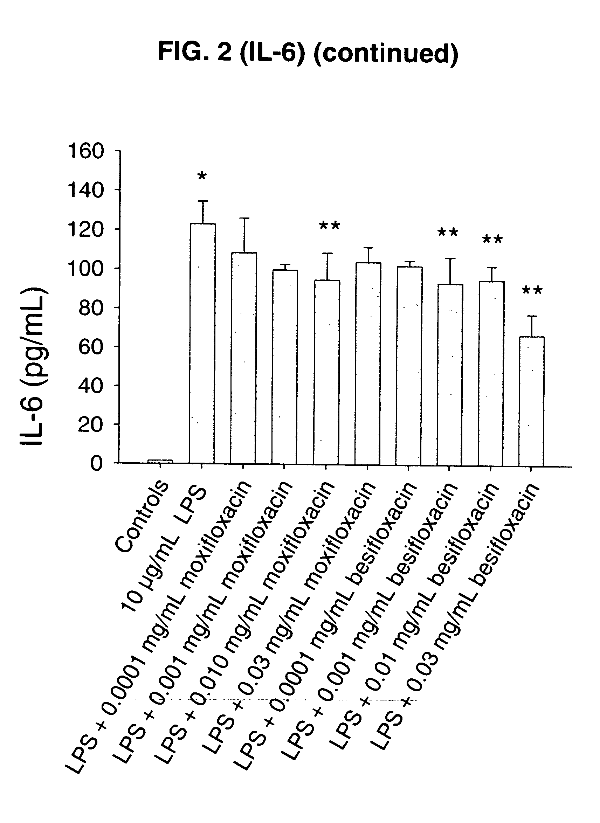

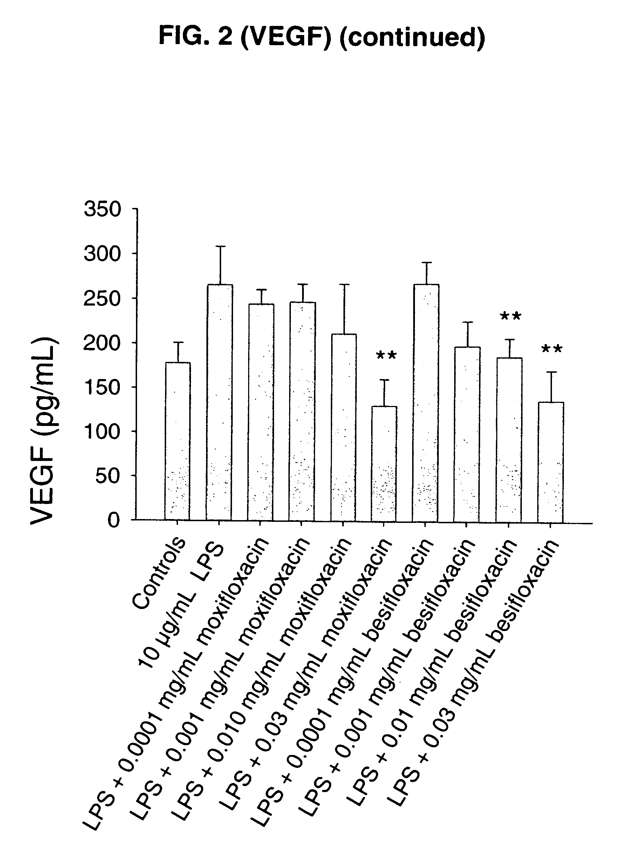

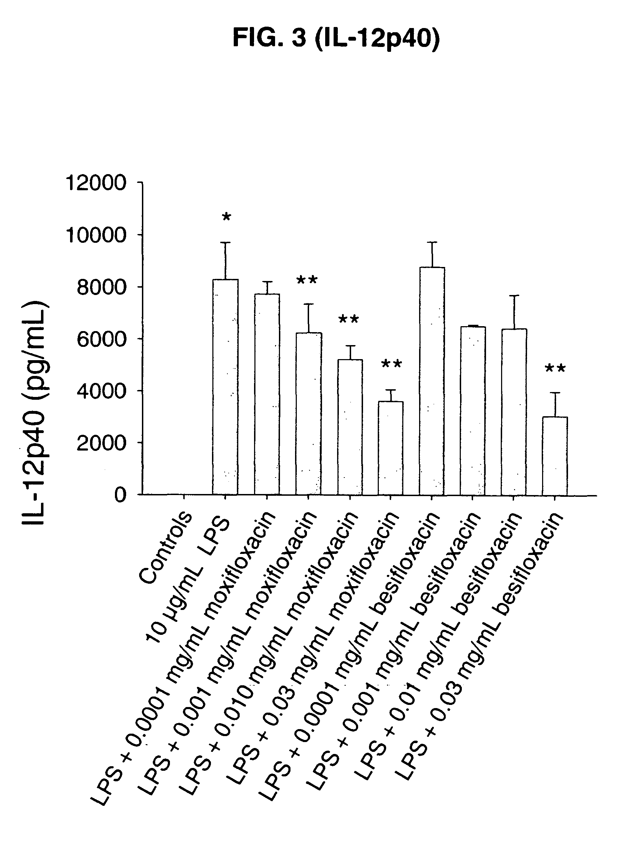

The cytokines detected in this study were divisible into four different response groups. The first group includes those cytokines for which these fluoroquinolones had no significant efficacy (RANTES and fractalkine). The second group of cytokines includes GM-CSF, IL-1β, IL-8, IP-10, MCP-1, and MIP-1α. For these cytokines, both moxifloxacin and compound having Formula IV (labeled as BOL-303224-A in the figures) had comparable effects after LPS stimulation (Figure 1). The third group of cytokines, including G-CSF, IL-1α, IL-1ra, IL-6, and VEGF are those for which compound having Formula IV demonstrated better potency than moxifloxacin (Figure 2). Finally, the fourth group of cytokines are those for which moxifloxacin was more potent than compound having Formula IV, and consists of only IL-12p40 (Figure 3).

With the compound having Formula IV, significant cytokine inhibitory effects were observed at very low concentrations. For example, a significant inhibitory effect of compound having Formula IV was seen at as low as 100 ng/mL on IL-1α, and at 1000 ng/mL on G-CSF, IL-1ra, and IL-6. These concentrations are well below predicted ocular concentrations following topical administration (K.W. Ward et al., J. Ocul. Pharmacol. Ther., Vol. 23, 243-256 (2007)). Therefore, clinical benefits resulting from this cytokine inhibition profile can be obtained.

### TESTING 2: Evaluation of the Efficacy of Four Antibiotic Formulations in a Prophylactic Endophthalmitis Model in New Zealand White Rabbits

### Introduction

The objective of this study was to evaluate the efficacy of four antibiotic formulations in treating bacterial endophthalmitis in New Zealand White rabbits.

### Materials and Methods

### Test Articles

Four antibiotic formulations were used in this study and identified as follows:
- BOL-303224-A (0.6% suspension)
- Quixin^{®} (0.5% Levofloxacin)
- Vigamox^{®} (0.5% Moxifloxacin)
- Zymar^{®} (0.3% Gatifloxacin)

The test articles were stored at room temperature and used as provided. A material safety data sheet (MSDS) or package insert with relevant safety information was provided for each test article. Normal saline was used as a negative control article and administered in the same manner as the antibiotic formulations. Further information on the test and control articles is shown in Table 3.

**Table 3**

| Article | Description |
|---|---|
| BOL-303224-A | 0.6% suspension (6 mg/mL): Bausch & Lomb, Lot No. 037931 |
| Quixin^{®} | Levofloxacin ophthalmic solution, 0.5% (5 mg/mL); Vistakon^{®} Pharmaceuticals, LLC. Lot No. 108020, exp. 04/09 |
| Vigamox^{®} | Moxifloxacin hydrochloride ophthalmic solution, 0.5% as base (5 mg/mL): Alcon Laboratories. Inc., Lot No. 122453F. exp. 02/09 |
| Zymar^{®} | Gatifloxacin ophthalmic solution, 0.3% (3 mg/mL); Allergan. Inc., Lot No. 48125, exp. 12/08, and Lot No. 48517, exp. 01/09. |
| Normal Saline | 0.9% Sodium Chloride Injection USP; B. Braun Medical Inc., Lot No. J6L012, exp. 09/08, and Lot No. J7D025. exp. 04/09 |

### Bacterial Inoculum

Methicillin-resistant *Staphylococcus aureus* (*S. aureus*), strain ATCC 33591 (MicroBiologics Power™ Microorganisms, Lot No. 496431, exp. 01/09, count/pellet: 2.6 x 10⁸), was used for induction of bacterial endophthalmitis. *S. aureus* was supplied as lyophilized pellets and stored refrigerated (2-8°C) prior to hydration. An MSDS was supplied with *S*. *aureus.* Buffered water (APHA) (Remel Corp., Lot No. 472492, exp. 9/11/07, Lot No. 540843, exp. 4/18/08) was used as a hydration fluid. Balanced salt solution (BSS) (B. Braun Medical, Lot No. J6N011, exp. 10/08) was used to prepare suspensions of *S*. *aureus* for inoculation.

A suspension of *S. aureus* was prepared on each day of inoculation as follows: The lyophilized *S. aureus* pellets and hydration fluid were brought to room temperature. Two to three pellets were placed with sterile forceps into 10 mL of hydration fluid in a vial. The vial was capped and incubated at 34-38°C for 30 minutes to assure complete hydration. After incubation, the hydrated material was vortexed to achieve a homogeneous suspension and equal distribution of the organism. This suspension was used as an inoculum on the day of preparation.

Each inoculum was enumerated for colony-forming units (CFU) as follows: Serial 1:10 (initial volume:final volume) dilutions were prepared with BSS, and duplicate pour-plates of the dilutions (1 mL/plate) were made with tryptic soy agar (TSA). The plates were incubated at 30-35°C for 29-47 hours and then counted. The resulting concentrations of the inoculums were 3.5 x 10⁷ CFU/mL (for Groups A-C), 2.9 x 10⁷ CFU/mL (for Groups D-F), and 3.3 x 10⁷ CFU/mL (for Groups G-I).

The dose volume (25 µL) of each inoculum was enumerated as follows: 0.025 mL of inoculum was placed into 9.975 mL of BSS. Serial 1:10 dilutions were prepared with BSS, and duplicate pour-plates of the dilutions (1 mL/plate) were made with TSA. The plates were incubated at 30-35°C for 29-47 hours and then counted. The resulting concentrations of the inoculums were 2.5 x 10⁵ CFU/dose (for Groups A-C), 7.5 x 10⁵ CFU/dose (for Groups D-F), and 4.1 x 10⁵ CFU/dose (for Groups G-I).

The following deviations from protocol occurred during enumeration of inoculums: For the first dilution, 0.025 mL of inoculum was placed into 9.975 mL of BSS; the protocol specified that 1.0 mL of inoculum would be placed into 1.45 mL of BSS. The prepared plates were incubated at 30-35°C; the protocol specified that plates would be incubated at 34-38°C. These deviations had no effect on the outcome of the study.

### Test System

### Animals

Fifty-one female New Zealand White rabbits were obtained from The Rabbit Source (Ramona, California). Animals were 9-15 weeks old and weighed 1.6-2.5 kg at the time of dosing. The protocol specified that animals would weigh at least 2.0-3.0 kg at the time of dosing, but eight animals in Groups D-F weighed less than 2.0 kg. This deviation had no effect on the outcome of the study. Animals were identified by ear tags and cage cards.

### Animal Husbandry

Upon arrival, animals were examined to ensure that they were healthy and quarantined for 10 days before placement on study. At the end of the quarantine period, animals were again examined for general health parameters and for any anatomical ophthalmic abnormalities.

Animals were housed in individual, hanging, stainless steel cages. Housing and sanitation were performed according to internal operating procedure.

Animals were provided Teklad Certified Global High Fiber Rabbit Diet daily. Diet certification and analysis were provided by the vendor, Harlan Teklad. No analyses outside those provided by the manufacturer were performed. Animals were provided tap water *ad libitum.* No contaminants were known to exist in the water and no additional analyses outside those provided by the local water district and as specified in internal operating procedure were performed. Environmental parameters were monitored according to internal operating procedure. The study room temperature was 70-73°F with 57-86% relative humidity.

### Pre-Treatment Examinations

Prior to placement on study, each animal underwent a pre-treatment ophthalmic examination (slit lamp and indirect ophthalmoscopy). Observations were scored according to the McDonald Shadduck system and recorded using a standardized data collection sheet. Acceptance criteria for placement on study were as follows: Scores of ≤ 1 for conjunctival congestion and swelling; scores of 0 or 3 for pupillary response (indicating that pupil response was normal (score = 0) or that pupils were dilated with a mydriatic agent prior to ophthalmoscopy (score = 3)); scores of 0 for all other observation variables.

### Treatment Groups

Treatment groups are described in Table T2-I. The study was conducted in three phases as follows: Animals in Groups A-C (BOL-303224-A, Zymar^{®}, and saline) were inoculated first; animals in Groups D-F (Quixin^{®}, Vigamox^{®}, and untreated) were inoculated eight days later; and animals in Groups G-I (Quixin^{®}, Vigamox^{®}, and saline) were inoculated thirty-three days after the first group.

Prior to treatment in each phase, animals were weighed and randomly assigned to the groups scheduled for treatment with one exception: eight days after the first group was inoculated, Group F animals (untreated controls) were added to the study after animals were randomized to Groups D and E (Quixin^{®} and Vigamox^{®}). The protocol indicated that animals would be weighed and randomized to treatment groups in each phase. As the weights of Group F animals were similar to the weights of Group D and E animals, this deviation had no effect on the outcome of the study. Animals were randomized to treatment groups according to modified Latin squares.

### Antibiotic Dosing

The right eyes of animals in Groups A-E and G-I were treated with the appropriate article (antibiotic agent or saline) before and after intracameral inoculation. The article was topically administered via positive displacement pipette at a volume of 50 µL per dose. Each right eye received four doses of the article at 15-minute intervals prior to inoculation (at -60, -45, -30, and -15 minutes) and five doses of the article at 6-hour intervals following inoculation (immediately post-inoculation and at 6, 12, 18, and 24 hours). The time of each dose administration was recorded. The right eyes of Group F animals remained untreated before and after inoculation.

The protocol specified that doses of antibiotic agents or saline would be given within the following time ranges: ± 3 minutes of pre-inoculation intervals; immediately post-inoculation (no range); and ± 5 minutes of 6-hour or later post-inoculation intervals. The actual time ranges at some intervals were larger than those specified. Most doses were given within the following ranges: ± 4 minutes of pre-inoculation intervals; immediately to 5 minutes post-inoculation; ± 5 minutes of the 6-hour and 24-hour post-inoculation intervals; and ± 30 minutes of the 12-hour and 18-hour post-inoculation intervals. For dosing intervals before and immediately after inoculation, the time ranges were slightly increased since the inoculation and antibiotic dosing were performed in separate rooms by different personnel to maintain sterility. The deviations in dosing-time ranges had no apparent effect on the outcome of the study.

### Fasting

Animals in Groups A-E and G-I were fasted at least one hour prior to intracameral dosing. The start time of the fast and the time of intracameral dosing were recorded. Animals in Group F were not fasted prior to dosing. The protocol specified that all animals would be fasted at least two hours prior to intracameral dosing. This deviation had no effect on the outcome of the study.

### Anesthesia

Prior to intracameral dosing, animals were weighed and anesthetized with an intravenous injection of a ketamine/xylazine cocktail (77 mg/mL ketamine, 23 mg/mL xylazine) at dose of 0.1 mg/kg.

### Eye Preparation

Proparacaine hydrochloride 0.5% (1-2 drops) was delivered to each right eye prior to intracameral dosing.

### Intracameral Dosing Procedure

On Day 1, each animal received a 25-µL, intracameral injection of S. *aureus* inoculum in the right eye. Intracameral injections were given using a Hamilton syringe with an attached 30-gauge x 1/2-inch needle. The protocol specified that intracameral injections would be given using a 30-gauge x 5/8-inch needle. It also specified that collected data would include dosing syringe weights, but the syringes were not weighed during injections. These deviations had no effect on the outcome of the study. The intracameral injection was made through the limbus into the central anterior chamber.

Immediate post-injection tamponade was applied with sterile cotton swabs or conjunctival compression over the injection site. A small amount of leakage was noted after 14 injections as follows: Group A, Nos. 2983 and 2969; Group B, Nos. 2967 and 2953; Group D, Nos. 3326, 3329, 3340, and 3334; Group E, No. 3091; Group F, Nos. 3078 and 3088; Group G, Nos. 3524 and 3552; Group H, No. 3537. The time of each injection was recorded. To maintain sterility, inoculation and antibiotic dosing were performed in separate rooms by different personnel.

### Mortality/Morbidity

Animals were observed for mortality/morbidity twice daily.

### Body Weights

Animals were weighed at randomization and prior to intracameral dosing.

### Ophthalmic Observations

Slit lamp ophthalmic observations (including observations of the conjunctiva, cornea, and iris) and indirect ophthalmoscopy (observation of the posterior segment) were performed on both eyes of each animal on Day 2, after the final doses of antibiotic agent or saline were administered. Eyes of Group D-I animals were also observed for pupil response, aqueous flare, cellular flare, and lens opacity. The protocol did not specify that eyes would be observed for pupil response, aqueous flare, cellular flare, and lens opacity. This deviation provided more data for evaluation, and it had no adverse effect on the outcome of the study. Ocular findings were scored using a severity scale of 0 to 3 or 0 to 4 for each described symptom (blepharitis, iritis, conjunctivitis, corneal edema, and corneal infiltrates). The protocol specified that ocular findings would be scored using a severity scale of 0 to 3 for each symptom, but for some ocular symptoms, findings were scored using a scale of 0 to 4. This deviation had no effect on the outcome of the study. The highest possible total score per eye was 27 (excluding scores for pupil response, aqueous flare, cellular flare, and lens opacity). The scoring system for the ophthalmic examinations and clinical evaluation of the anterior and posterior segments is shown in Table T2-2.

### Euthanasia

Following completion of the 24-hour clinical ophthalmic examination, animals were euthanized with an intravenous injection of commercial euthanasia solution. Euthanasia was performed according to established internal operating procedure.

### Necropsy

After euthanasia, the aqueous and vitreous humors were aseptically collected from each right eye to determine numbers of viable bacteria in these tissues. The aqueous and vitreous humor samples were collected using a 30-gauge ½-inch needle and a 21-gauge 1-inch needle, respectively. The volumes of the collected samples were recorded. Each vitreous humor sample was liquefied by passing it through a 25-gauge needle three times (performed in a biological safety hood). Blood was observed in one vitreous humor sample (Group E, No. 3336).

### Bacterial Enumeration

Bacterial counts in the aqueous and vitreous humor samples were determined as follows: For each sample, 10-fold dilutions (initial volume:final volume = 1:10, 1:100, 1:1000, and 1:10000) were made with sterile phosphate buffer. Each dilution was plated in duplicate (mL/plate) on TSA, and the plates were incubated for 46-48 hours at 30-35°C. The colonies on each plate were counted, and the counts of duplicate plates were averaged. The dilution with less than 300 bacterial colonies per plate was used to calculate the bacterial number in each sample. The number of viable *S. aureus* organisms (CFU) was expressed as a base 10 logarithm. Sample plates with unusual bacterial counts were subjected to species identification using a Vitek (BioMerieux) automated microbial identification system.

The following deviations from protocol occurred during enumeration: The sample dilution ratios used for plating were 1:10, 1:100, 1:1000, and 1:10000; the protocol specified that the ratios would be 1:1, 1:10, 1:100, and 1:1000. The TSA used in plating was supplied by Remel Corp; the protocol specified that the TSA would be supplied by Difco. Sample plates were incubated for 46-48 hours at 30-35°C; the protocol specified that sample plates would be incubated for 48 hours at 34-38°C. Sample plates with unusual bacterial counts were subjected to species identification; the protocol did not indicate that microbial identification would be performed. These deviations had no effect on the outcome of the study.

### Statistical Analysis

Descriptive statistics (mean and standard deviation) were calculated for total ophthalmic severity scores of each treatment group. Remaining data were evaluated by inspection only.

### Animal Welfare Statement

This study was performed to evaluate the efficacy of the test articles in treating bacterial endophthalmitis. Alternatives to performing this study were explored; however, to properly evaluate the efficacy of the test articles, a whole-body test system was required. This study complied with all internal animal welfare policies and was approved by the Institutional Animal Care and Use Committee.

### Results

### Mortality

There was no unscheduled mortality of any animal on study.

### Ophthalmic Observations

Mean total ophthalmic severity scores are presented in Table T2-3. General differences between groups were as follows: Eyes treated with BOL-303224-A (Group B) had lower total scores than eyes treated with Zymar^{®} (Group C), Quixin^{®} (Groups D and H), Vigamox^{®} (Groups E and I), saline (Groups A and G), or untreated (Group F). Differences in total scores between the Zymar^{®}, Quixin^{®}, Vigamox^{®}, and saline/untreated groups were not as clear, mainly due to variability within these groups. Eyes inoculated with 7.5 x 10⁵ CFU appeared to have higher total scores than eyes inoculated with 4.1 x 10⁵ CFU and administered similar treatments (Quixin^{®}, Vigamox^{®}, or saline/untreated).

Ophthalmic observations of individual animals are presented in Table T2-4. Observations of untreated left eyes are not shown because all left eyes appeared normal. Signs of inflammation were observed in all right eyes; anomalies commonly seen among all groups included conjunctival congestion and swelling; corneal lesions (without pannus); aqueous and cellular flare; iris involvement; and fibrin in the anterior chamber. Conjunctival discharge was frequently seen in right eyes of all groups except Group B (BOL-303224-A), in which no conjunctival discharge was observed. Poor pupil response was frequently seen in right eyes of all groups except Groups G-I (4.1 x 10⁵ CFU-inoculation groups), in which pupil response was mostly normal. The posterior segment of the eye was not visible in 17 of 18 eyes inoculated with 2.5 x 10⁵ CFU (Groups A-C), nor in 14 of 15 eyes inoculated with 7.5 x 10⁵ CFU (Groups D-F), but it was visible in 14 of 18 eyes inoculated with 4.1 x 10⁵ CFU (Groups G-I).

### Bacterial Enumeration

Bacterial counts in aqueous and vitreous humor samples are shown in Table T2-5. Viable bacteria were found in aqueous humor samples as follows: All 6 saline-treated eyes inoculated with 2.5 x 10⁵ CFU (Group A); 2 of 6 saline-treated eyes inoculated with 4.1 x 10⁻⁵ CFU (Group G); 2 of 3 untreated eyes (Group F); and 2 of 6 Quixin^{®}-treated eyes inoculated with 4.1 x 10⁵ CFU (Group H). Of the samples from the saline-treated eyes, one (Group G, No. 3551) had a calculated bacterial count exceeding 3 x 10⁶ CFU; this was higher than the count in the inoculum. The plate-colonies grown from this sample were identified as two contaminating species *(Enterobacter cloacae* and *Enterobacter aerogenes).* Excluding the contaminated sample, the highest bacterial counts, 195 and 135 (log₁₀(CFU) = 2.29 and 2.13, respectively), were found in aqueous humor samples from two untreated eyes (Group F). For comparison, the bacterial count (log)₁₀(CFU)) injected in these eyes was 5.88.

No viable bacteria were found in aqueous humor samples from the remaining saline-treated, untreated, or Quixin^{®}-treated eyes, nor in any eyes treated with 0.6% BOL-303224-A (Group B), Zymar^{®} (Group C), or Vigamox^{®} (Groups E and I). No viable bacteria were found in any vitreous humor samples.

### Conclusion

The objective of this study was to evaluate the efficacy of four antibiotic formulations in treating bacterial endophthalmitis in New Zealand White rabbits. In conclusion, intracameral injection of 2.5 x 10⁵ to 7.5 x 10⁵ CFU *S*. *aureus* in rabbit eyes induced endophthalmitis within 24 hours of inoculation as indicated by ophthalmic findings. The ophthalmic findings suggested that BOL-303224-A (compound having Formula IV) controlled ocular inflammation associated with endophthalmitis, especially conjunctival discharge, more effectively than the other commercial antibiotic products or saline/no treatment. Vitreous humor samples collected 24 hours post-inoculation contained no viable bacteria, whether or not the eyes received antibiotic treatment. Most aqueous humor samples collected 24 hours post-inoculation contained no viable bacteria, including samples from five eyes that received no antibiotic treatment. For nine eyes that received no antibiotic treatment, the aqueous humor samples contained viable *S*. *aureus* but at substantially reduced populations. Some reduction in bacterial counts could be attributed to the rabbit immune system itself and to the bacterial species selected, *S*. *aureus,* which might not flourish in an environment that is more anaerobic in nature.

**Table T2-1**

| Treatment Groups | | | | | | |
|---|---|---|---|---|---|---|
| Group | No. | Topical Antibiotic Treatment (Right Eye) | Dose Volume | Bacteria Dosing (Right eye) | *S*. *aureus* ATCC 33591 Dose Volume | Necropsy |
| A | 6 | Saline | 50 µL | Intracameral | 25 µL | Day 2 |
| B | 6 | 0.6% BOL-303224-A | 50 µL | Intracameral | 25 µL | Day 2 |
| C | 6 | Zymar^{®} (0.3% Gatifloxacin) | 50 µL | Intracameral | 25 µL | Day 2 |
| D | 6 | Quixin^{®} (0.5% Levofloxacin) | 50 µL | Intracameral | 25 µL | Day 2 |
| E | 6 | Vigamox^{®} (0.5% Moxifloxacin) | 50 µL | Intracameral | 25 µL | Day 2 |
| F | 3 | Untreated | N/A | Intracameral | 25 µL | Day 2 |
| G | 6 | Saline | 50 µL | Intracameral | 25 µL | Day 2 |
| H | 6 | Quixin^{®} (0.5% Levofloxacin) | 50 µL | Intracameral | 25 µL | Day 2 |
| I | 6 | Vigamox^{®} (0.5% Moxifloxacin) | 50 µL | Intracameral | 25 µL | Day 2 |

**Table T2-2**

| Clinical Ophthalmic Scoring System | |
|---|---|
| ANTERIOR SEGMENT: | |
| Conjunctival Congestion | |
| 0 = | Normal. May appear blanched to reddish pink without perilimbal injection (except at 12:00 and 6:00 positions) with vessels of the palpebral and bulbar conjunctiva easily observed. |
| 1 = | A flushed. reddish color predominantly confined to the palpebral conjunctiva with some perilimbal injection but primarily confined to the lower and upper parts of the eye from the 4:00 to 7:00 and 11:00 to 1:00 positions. |
| 2 = | Bright red color of the palpebral conjunctiva with accompanying perilimbal injection covering at least 75% of the circumference of the perilimbal region. |
| 3 = | Dark, beefy red color with congestion of both the bulbar and palpebral conjunctiva along with pronounced perilimbal injection and the presence of petechia on the conjunctiva. The petechia generally predominate along the nictitating membrane and upper palpebral conjunctiva. |

| Conjunctival Swelling | |
|---|---|
| 0 = | Normal or no swelling of the conjunctival tissue |
| 1 = | Swelling above normal without eversion of the eyelids (easily discerned by noting upper and lower eyelids are positioned as in the normal eye); swelling generally starts in the lower cul-de-sac near the inner canthus. |
| 2 = | Swelling with misalignment of the normal approximation of the lower and upper eyelids; primarily confined to the upper eyelid so that in the initial stages, the misapproximation of the eyelids begins by partial eversion of the upper eyelid. In this stage the swelling is confined generally to the upper eyelid with some swelling in the lower cul-de-sac. |
| 3 = | Swelling definite with partial eversion of the upper and lower eyelids essentially equivalent. This can be easily observed by looking at the animal head-on and noting the position of the eyelids: if the eye margins do not meet, eversion has occurred. |
| 4 = | Eversion of the upper eyelid is pronounced with less pronounced eversion of the lower eyelid. It is difficult to retract the lids and observe the perilimbal region. |

| Conjunctival Discharge | |
|---|---|
| Discharge is defined as a whitish. gray precipitate. | |
| 0 = | Normal. no discharge. |
| 1 = | Discharge above normal and present on the inner portion of the eye but not on the lids or hairs of the eyelids. |
| 2 = | Discharge is abundant, easily observed and has collected on the lids and hairs of the eyelids. |
| 3 = | Discharge has been flowing over the eyelids so as to wet the hairs substantially on the skin around the eye. |

| Iris Involvement | |
|---|---|
| 0 = | Normal iris without any hyperemia of the blood vessels. |
| 1 = | Minimal injection of the secondary vessels but not tertiary vessels. Generally uniform but may be of greater intensity at the 12:00 to 1:00 or 6:00 position. If confined to this area. the tertiary vessels must be substantially hyperemic. |
| 2 = | Moderate injection of the secondary and tertiary vessels with slight swelling of the iris stroma (the iris surface appears slightly rugose, usually most predominant near the 3:00 and 9:00 positions). |
| 3 = | Marked injection of the secondary and tertiary vessels with marked swelling of the iris stroma. The iris appears rugose; may be accompanied by hemorrhage (hyphema) in the anterior chamber. |

| Cornea | |
|---|---|
| 0 = | Normal Cornea. |
| 1 = | Some loss of transparency. Only the epithelium and/or the anterior half of the stoma are involved. The underlying structures are clearly visible although some cloudiness may be readily apparent. |
| 2 = | Moderate loss of transparency. The cloudiness extends all the way to the endothelium. With diffuse illumination, underlying structures are clearly visible although there may be some loss of detail. |
| 3 = | Involvement of the entire thickness of the stroma. With diffuse illumination, the underlying structures are just barely visible (can still observe flare, iris. pupil response, lens). |
| 4 = | Involvement of entire thickness of the stroma. With diffuse illumination, the underlying structures cannot be seen. |

| Surface Area of Cornea Involvement | |
|---|---|
| 0 = | Normal |
| 1 = | 1-25% area of stromal cloudiness. |
| 2 = | 26-50% |
| 3= | 51-75% |
| 4 = | 76-100% |

| Pannus (Vascularization of Cornea) | |
|---|---|
| 0= | No pannus |
| 1 = | Vascularization present but vessels have not invaded the entire corneal circumference. |
| 2 = | Vessels have invaded 2 mm or more around entire corneal surface. |

| POSTERIOR SEGMENT: | |
|---|---|
| 0 = | Normal eye without vitreous haze. |
| 1 = | Vitreous haze allowing observation of the optic nerve and retinal vessels. |
| 2 = | Vitreous haze still allowing observation of major vessels and optic nerve with difficulty. |
| 3 = | Vitreous haze allowing observation of the boundaries of the optic nerve only, its boundaries being blurred. |
| 4 = | Vitreous haze preventing observation of the optic nerve. |

| OTHER VARIABLES: (Scores excluded from total severity score) | |
|---|---|
| Pupillary Response | |
| 0 = | Normal pupil response. |
| 1 = | Sluggish or incomplete pupil response. |
| 2 = | No pupil response. |
| 3 = | No pupil response due to pharmacological blockage. |

| Aqueous Flare | |
|---|---|
| 0= | None |
| 1= | 1+ |
| 2= | 2+ |
| 3= | 3+ |
| 4= | 4+ (fibrin) |

| Cellular Flare | |
|---|---|
| 0= | None |
| 1= | 1+ |
| 2= | 2+ |
| 3= | 3+ |
| 4= | 4+ |

| Lens (Observe lens for cataract) | |
|---|---|
| 0= | Lens clear. |
| 1 = | Anterior (cortical/capsular). |
| 2 = | Nuclear. |
| 3 = | Posterior (cortical/capsular). |
| 4 = | Equatorial. |

**Table T2-3**

| Mean Total Ocular Severity Scores | | | | |
|---|---|---|---|---|
| | Topical Antibiotic Treatment (Right Eye) | Total Severity Score | | |
| Group | | Mean | Std.Dev. | N |
| A | Saline | 10.0 | 1.4 | 6 |
| B | 0.6% BOL-303224-A | 6.7 | 1.4 | 6 |
| C | Zymar^{®} (0.3% Gatifloxacin) | 9.0 | 1.1 | 6 |
| D | Quixin^{®} (0.5% Levofloxacin) | 10.0 | 3.7 | 6 |
| E | Vigamox^{®} (0.5% Moxifloxacin) | 10.8 | 4.0 | 6 |
| F | Untreated | 13.0 | 1.7 | 3 |
| G | Saline | 9.7 | 3.1 | 6 |
| H | Quixin^{®} (0.5% Levofloxacin) | 8.5 | 1.4 | 6 |
| I | Vigamox^{®} (0.5% Moxifloxacin) | 8.3 | 1.8 | 6 |

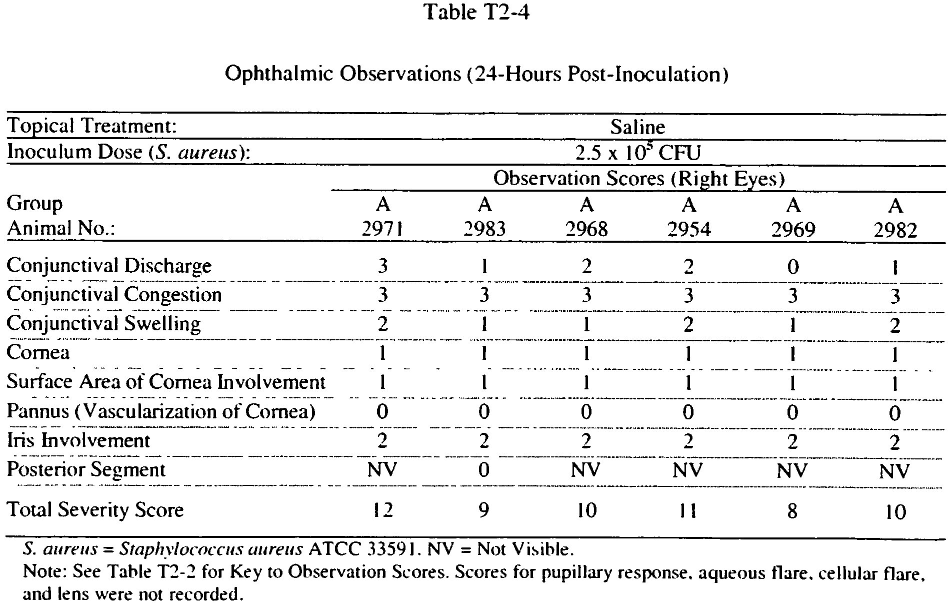

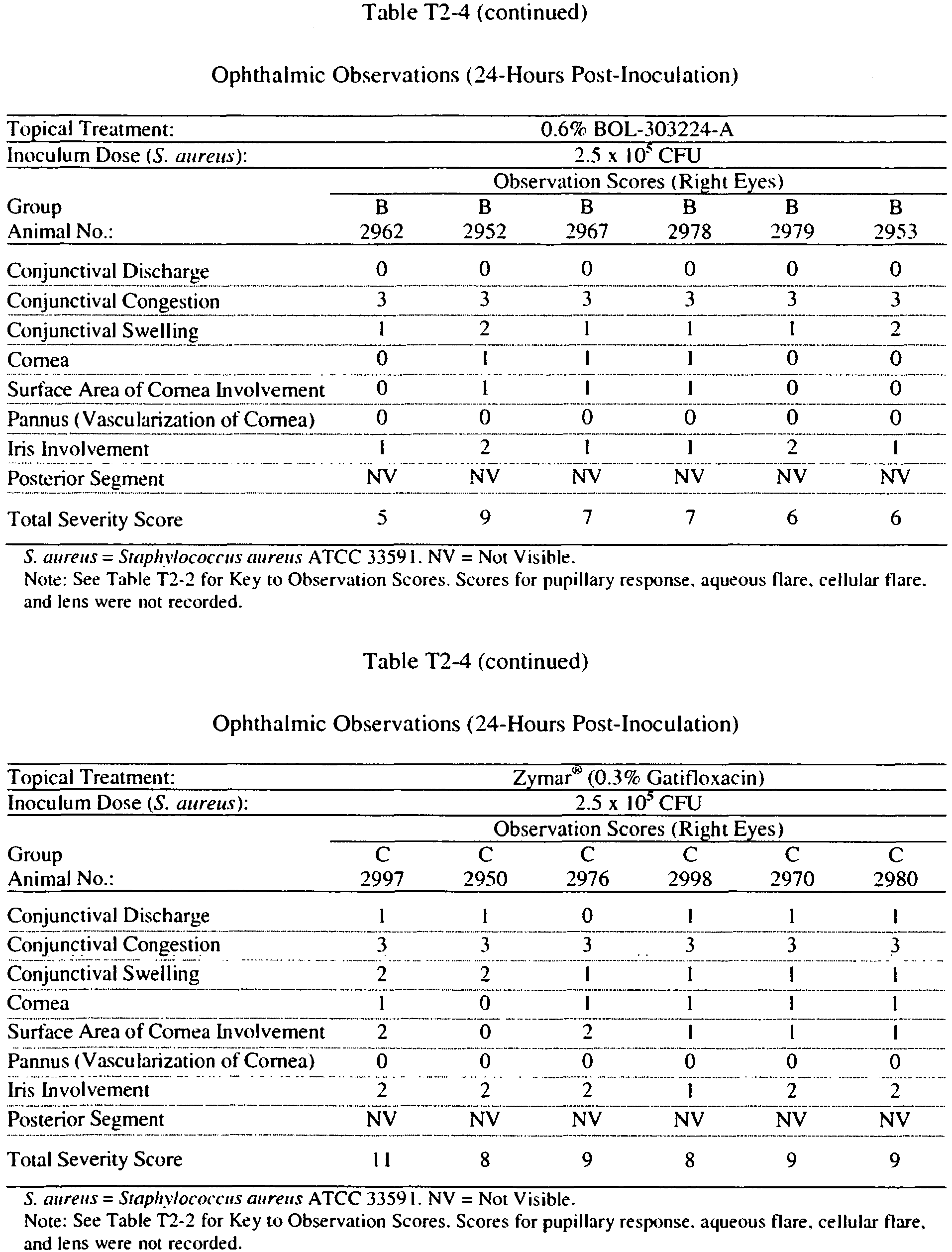

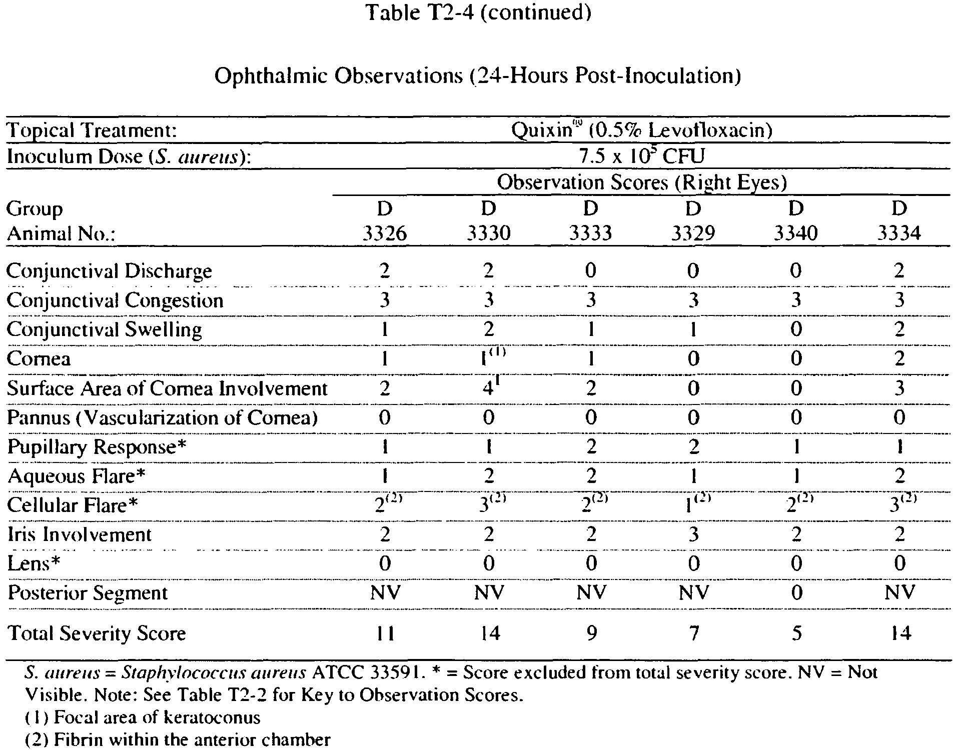

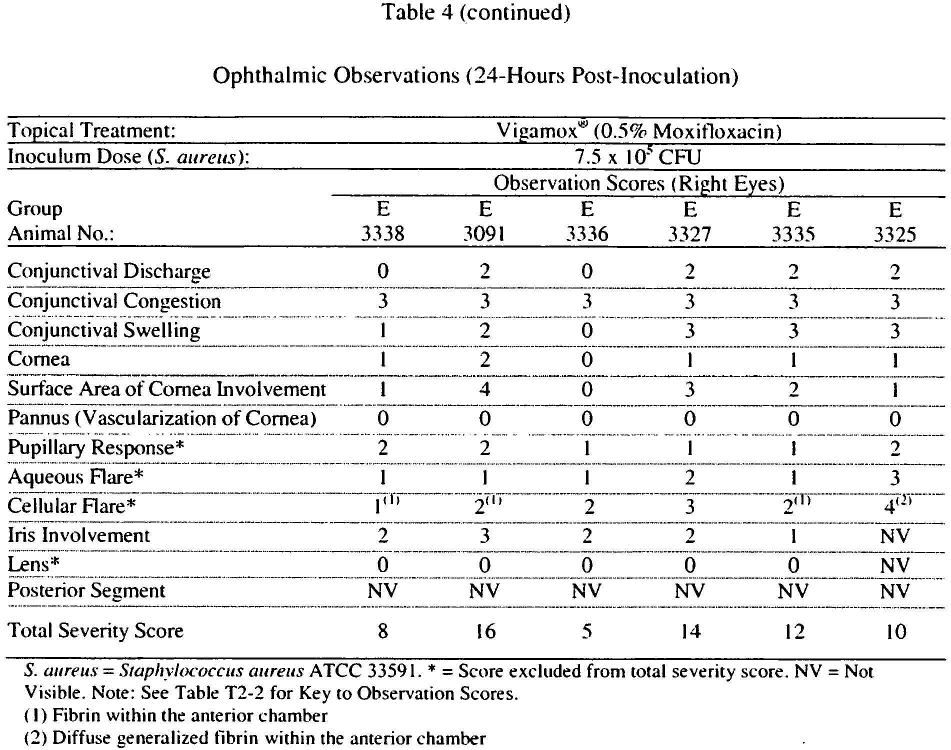

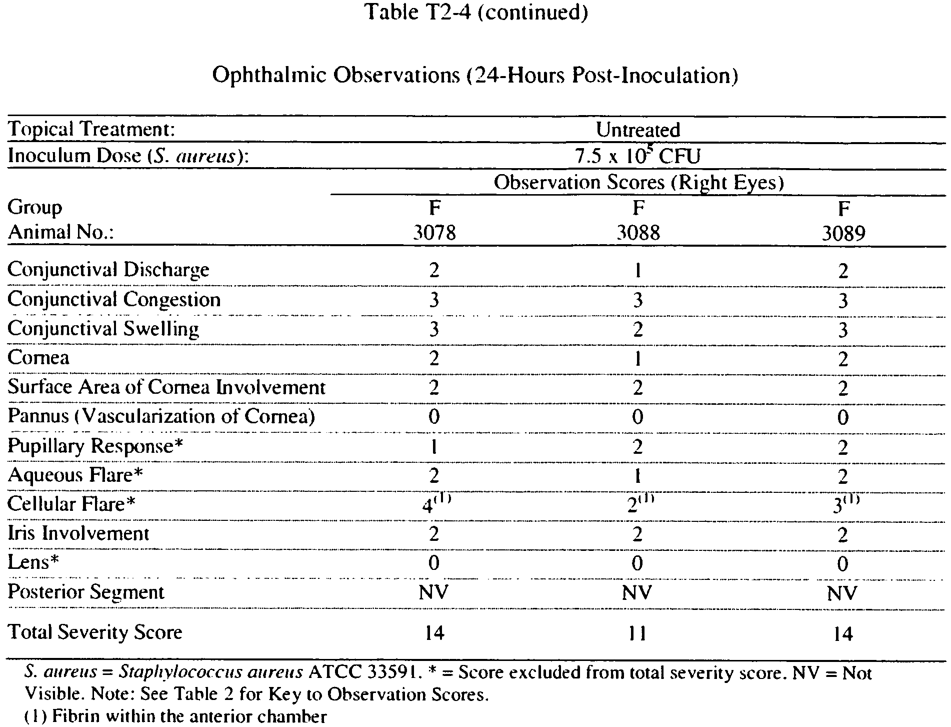

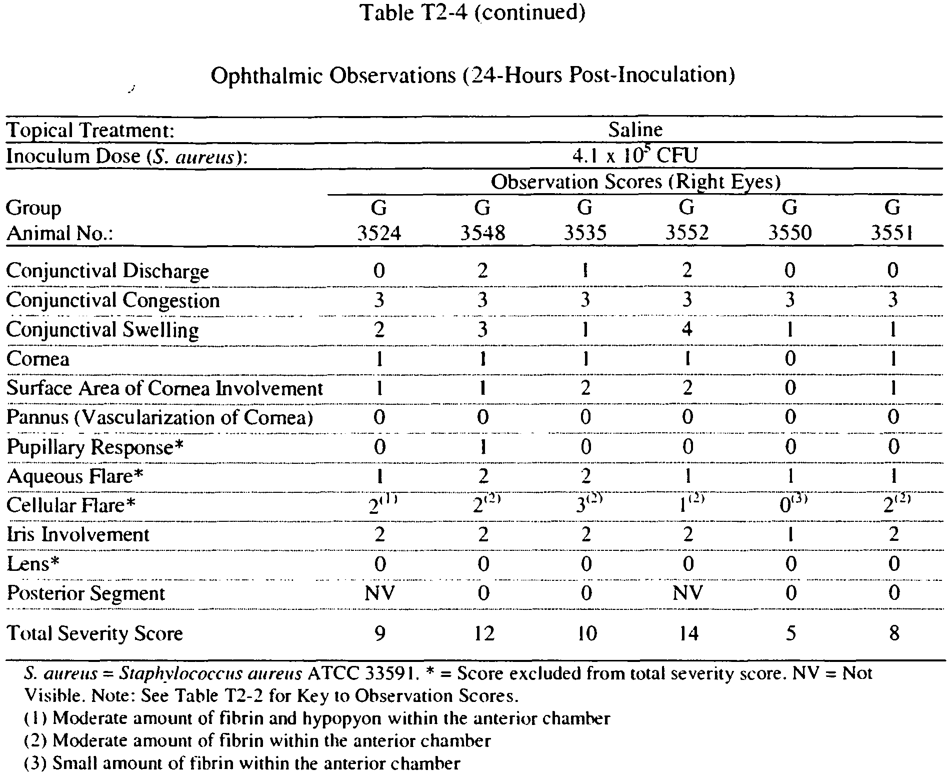

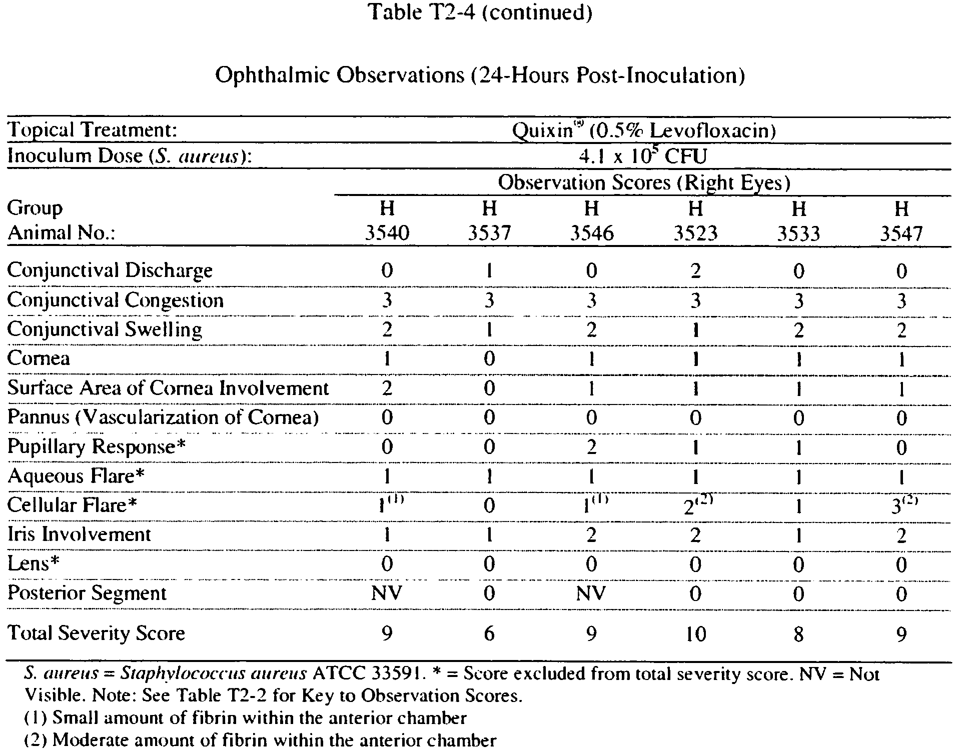

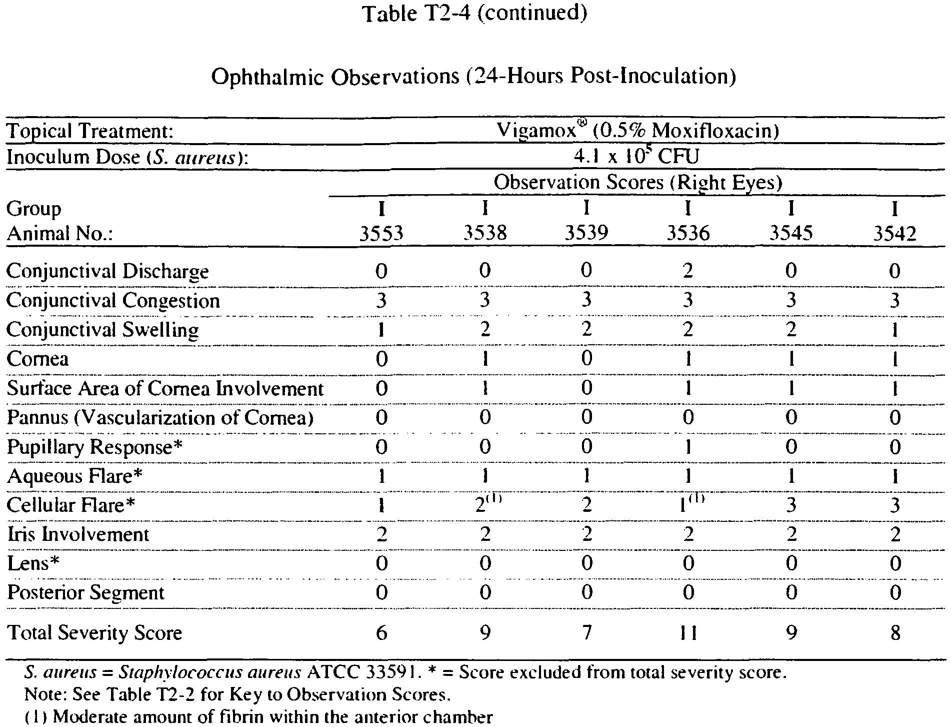

**Table T2-5**

| Bacterial Counts in Aqueous and Vitreous Humor Samples | | | | | | | | |
|---|---|---|---|---|---|---|---|---|
| | | | Counts of Viable Bacteria *(S. aureus)* Per Sample | | | | | |
| Group | Topical Treatment | Rabbit No. | Inoculum (25-µL) | | Aqueous Humor | | Vitreous Humor | |
| | | | CFU⁽¹⁾ | Log₁₀(CFU) | CFU⁽¹⁾ | Log₁₀(CFU) | CFU⁽¹⁾ | Log₁₀(CFU) |
| A | Saline | 2971 | 2.5 x10⁵ | 5.40 | 5 | 0.70 | 0 | N/A |
| | | 2983 | 2.5 x 10⁵ | 5.40 | 5 | 0.70 | 0 | N/A |
| | | 2968 | 2.5 x 10⁵ | 5.40 | 10 | 1.00 | 0 | N/A |
| | | 2954 | 2.5 x 10⁵ | 5.40 | 15 | 1.18 | 0 | N/A |
| | | 2969 | 2.5 x 10⁵ | 5.40 | 30 | 1.48 | 0 | N/A |
| | | 2982 | 2.5 x 10⁵ | 5.40 | 10 | 1.00 | 0 | N/A |
| B | 0.6% BOL-303224-A | 2962 | 2.5 x 10⁵ | 5.40 | 0 | N/A | 0 | N/A |
| | | 2952 | 2.5 x 10⁵ | 5.40 | 0 | N/A | 0 | N/A |
| | | 2967 | 2.5 x 10⁵ | 5.40 | 0 | N/A | 0 | N/A |
| | | 2978 | 2.5 x 10⁵ | 5.40 | 0 | N/A | 0 | N/A |
| | | 2979 | 2.5 x 10⁵ | 5.40 | 0 | N/A | 0 | N/A |
| | | 2953 | 2.5 x 10⁵ | 5.40 | 0 | N/A | 0 | N/A |
| C | Zymar^{®} (0.3% Gatifloxacin) | 2997 | 2.5 x 10⁵ | 5.40 | 0 | N/A | 0 | N/A |
| | | 2950 | 2.5 x 10⁵ | 5.40 | 0 | N/A | 0 | N/A |
| | | 2976 | 2.5 x 10⁵ | 5.40 | 0 | N/A | 0 | N/A |
| | | 2998 | 2.5 x 10⁵ | 5.40 | 0 | N/A | 0 | N/A |
| | | 2970 | 2.5 x 10⁵ | 5.40 | 0 | N/A | 0 | N/A |
| | | 2980 | 2.5 x 10⁵ | 5.40 | 0 | N/A | 0 | N/A |
| D | Quixin ^{®} (0.5% Levofloxacin) | 3326 | 7.5 x 10⁵ | 5.88 | 0 | N/A | 0 | N/A |
| | | 3330 | 7.5 x 10⁵ | 5.88 | 0 | N/A | 0 | N/A |
| | | 3333 | 7.5 x 10⁵ | 5.88 | 0 | N/A | 0 | N/A |
| | | 3329 | 7.5 x 10⁵ | 5.88 | 0 | N/A | 0 | N/A |
| | | 3340 | 7.5 x 10⁵ | 5.88 | 0 | N/A | 0 | N/A |
| | | 3334 | 7.5 x 10⁵ | 5.88 | 0 | N/A | 0 | N/A |
| E | Vigamox^{®} (0.5% Moxifloxacin) | 3338 | 7.5 x 10⁵ | 5.88 | 0 | N/A | 0 | N/A |
| | | 3091 | 7.5 x 10⁵ | 5.88 | 0 | N/A | 0 | N/A |
| | | 3336 | 7.5 x 10⁵ | 5.88 | 0 | N/A | 0 | N/A |
| | | 3327 | 7.5 x 10⁵ | 5.88 | 0 | N/A | 0 | N/A |
| | | 3335 | 7.5 x 10⁵ | 5.88 | 0 | N/A | 0 | N/A |
| | | 3325 | 7.5 x 10⁵ | 5.88 | 0 | N/A | 0 | N/A |
| F | Untreated | 3078 | 7.5 x 10⁵ | 5.88 | 0 | N/A | 0 | N/A |
| | | 3088 | 7.5 x 10⁵ | 5.88 | 195 | 2.29 | 0 | N/A |
| | | 3089 | 7.5 x 10⁵ | 5.88 | 135 | 2.13 | 0 | N/A |

| | Topical Treatment | Rabbit No. | Inoculum (25-µL) | | Aqueous Humor | | Vitreous Humor | |
|---|---|---|---|---|---|---|---|---|
| Group | | | CFU⁽¹⁾ | Log₁₀(CFU) | CFU⁽¹⁾ | Log₁₀(CFU) | CFU⁽¹⁾ | Log₁₀(CFU) |
| G | Saline | 3524 | 4.1 x 10⁵ | 5.61 | 0 | N/A | 0 | N/A |
| | | 3548 | 4.1 x 10⁵ | 5.61 | 0 | N/A | 0 | N/A |
| | | 3535 | 4.1 x 10⁵ | 5.61 | 0 | N/A | 0 | N/A |
| | | 3552 | 4.1 x 10⁵ | 5.61 | 20 | 1.30 | 0 | N/A |
| | | 3550 | 4.1 x 10⁵ | 5.61 | 0 | N/A | 0 | N/A |
| | | 3551 | 4.1 x 10⁵ | 5.61 | N/A⁽²⁾ | N/A⁽²⁾ | 0 | N/A |
| H | Quixin^{®} (0.5% Levofloxacin) | 3540 | 4.1 x 10⁵ | 5.61 | 0 | N/A | 0 | N/A |
| | | 3537 | 4.1 x 10⁵ | 5.61 | 0 | N/A | 0 | N/A |
| | | 3546 | 4.1 x 10⁵ | 5.61 | 5 | 0.70 | 0 | N/A |
| | | 3523 | 4.1 x 10⁵ | 5.61 | 0 | N/A | 0 | N/A |
| | | 3533 | 4.1 x 10⁵ | 5.61 | 5 | 0.70 | 0 | N/A |
| | | 3547 | 4.1 x 10⁵ | 5.61 | 0 | N/A | 0 | N/A |
| I | Vigamox^{®} (0.5% Moxifloxacin) | 3553 | 4.1 x 105 | 5.61 | 0 | N/A | 0 | N/A |
| | | 3538 | 4.1 x 10⁵ | 5.61 | 0 | N/A | 0 | N/A |
| | | 3539 | 4.1 x 10⁵ | 5.61 | 0 | N/A | 0 | N/A |
| | | 3536 | 4.1 x 10⁵ | 5.61 | 0 | N/A | 0 | N/A |
| | | 3545 | 4.1 x 10⁵ | 5.61 | 0 | N/A | 0 | N/A |
| | | 3542 | 4.1 x 10⁵ | 5.61 | 0 | N/A | 0 | N/A |

| | | | | | | | | |
|---|---|---|---|---|---|---|---|---|
| *S. aureus = Staphylococcus aureus* ATCC 33591. CFU = Colony Forming Units. N/A = Not applicable. (1) Average count of duplicate plates, adjusted for serial dilutions (2) Sample contaminated with Enterobacteria species | | | | | | | | |

A fluoroquinolone compound disclosed herein can be formulated into a pharmaceutical composition for topical, oral, subcutaneous, or systemic administration for the modulation of endophthalmitis or the treatment or control of an infection causing said endophthalmitis. Such a composition comprises a fluoroquinolone compound having Formula I, II. III, IV, V, VI, VII, or VIII or a salt thereof and a pharmaceutically acceptable carrier for the administration, as can be determined by a person having skill in the art of pharmaceutical formulation. For example, various pharmaceutically acceptable carriers known in the art can be used to formulate a solution, emulsion, suspension, dispersion, ointment, gel, capsule, or tablet. A fluoroquinolone compound having Formula I, II. III, IV, V, VI, VII, or VIII or a salt thereof is particularly suitable for a treatment or control of endophthalmitis caused by microorganisms or of non-infectious endophthalmitis. Such a fluoroquinolone or a salt thereof is formulated into a solution, ointment, emulsion, suspension, dispersion, or gel.

In one embodiment, a topical composition of the present invention comprises an aqueous solution or suspension. Typically, purified or deionized water is used. The pH of the composition is adjusted by adding any physiologically acceptable pH adjusting acids, bases, or buffers to within the range of 3 to 8.5 (or alternatively, or from 4 to 7.5, or from 4 to 6.5, or from 5 to 6.5). Examples of acids include acetic, boric, citric, lactic, phosphoric, hydrochloric, and the like, and examples of bases include sodium hydroxide, potassium hydroxide, tromethamine, THAM (trishydroxymethylaminomethane), and the like. Salts and buffers include citrate/dextrose, sodium bicarbonate, ammonium chloride and mixtures of the aforementioned acids and bases. pH buffers are introduced into the composition to maintain a stable pH and to improve product tolerance by the user. In some embodiments, the pH is in the range from about 4 to about 7.5. Biological buffers for various pHs are available, for example, from Sigma-Aldrich. A composition of the present invention can have a viscosity in the range from 5 to 100,000 centipoise ("cp") or mPa.s (or alternatively, from 10 to 50,000, or from 10 to 20,000, or from 10 to 10,000, or from 10 to 1,000, or from 100 to 10,000, or from 100 to 20,000, or from 100 to 50,000 or from 500 to 10,000, or from 500 to 20,000 cp).

In another embodiment, a topical composition of the present invention comprises an ointment, emulsion or cream (such as oil-in-water emulsion), or gel.

Ointments generally are prepared using either (1) an oleaginous base; i.e., one consisting of fixed oils or hydrocarbons, such as white petrolatum or mineral oil, or (2) an absorbent base; i.e., one consisting of an anhydrous substance or substances which can absorb water, for example anhydrous lanolin. Customarily, following formation of the base, whether oleaginous or absorbent, the active ingredient (compound) is added to an amount affording the desired concentration.

Creams are oil/water emulsions. They consist of an oil phase (internal phase), comprising typically fixed oils, hydrocarbons, and the like, such as waxes, petrolatum, mineral oil, and the like, and an aqueous phase (continuous phase), comprising water and any water-soluble substances, such as added salts. The two phases are stabilized by use of an emulsifying agent, for example, a surface active agent, such as sodium lauryl sulfate, hydrophilic colloids, such as acacia colloidal clays, veegum, and the like. Upon formation of the emulsion, the active ingredient (compound) customarily is added in an amount to achieve the desired concentration.

Gels comprise a base selected from an oleaginous base, water, or an emulsion-suspension base. To the base is added a gelling agent which forms a matrix in the base, increasing its viscosity. Examples of gelling agents are hydroxypropyl cellulose, acrylic acid polymers, and the like. Customarily, the active ingredient (compound) is added to the formulation at the desired concentration at a point preceding addition of the gelling agent.

The amount of a fluoroquinolone compound herein disclosed that is incorporated into a composition of the present invention is not critical; the concentration should be within a range sufficient to permit ready application of the formulation to the affected tissue area in an amount which will deliver the desired amount of compound to the desired treatment site and to provide the desired therapeutic effect. In some embodiments of the present invention, compositions comprise a fluoroquinolone in a concentration in a range from 0.0001% to 10% by weight (or alternatively, from 0.001 % to 5%, or from 0.01 % to 5%, or from 0.01 % to 2%, or from 0.01% to 1%, or from 0.01% to 0.7%, or from 0.01% to 0.5%, by weight).

Moreover, a topical composition of the present invention can contain one or more of the following: preservatives, surfactants, adjuvants including additional medicaments, antioxidants, tonicity adjusters, viscosity modifiers, and the like.

Preservatives may be used to inhibit microbial contamination of the product when it is dispensed in single or multidose containers, and can include: quaternary ammonium derivatives, (benzalkonium chloride; benzylammonium chloride, cetylmethyl ammonium bromide, cetylpyridinium chloride), benzethonium chloride, organomercury compounds (Thimerosal, phenylmercury acetate, phenylmercury nitrate), methyl and propyl p-hydroxybenzoates, betaphenylethyl alcohol, benzyl alcohol, phenylethyl alcohol, phenoxyethanol, and mixtures thereof. These compounds are used at effective concentrations, typically from 0.005% to 5% (by weight), depending on the preservative or preservatives selected. The amount of the preservative used should be enough so that the solution is physically stable: i.e., a precipitate is not formed, and antibacterially effective.

The solubility of the components, including a fluoroquinolone having Formula I, II, III, IV, V, VI, VII, or VIII, of the present compositions may be enhanced by a surfactant or other appropriate co-solvent in the composition or solubility enhancing agents like cyclodextrins such as hydroxypropyl, hydroxyethyl, glucosyl, maltosyl and maltotriosyl derivatives of α-, β-, and γ-cyclodextrin. In one embodiment, the composition comprises 0.1% to 20% hydroxypropyl- β-cyclodextrin; alternatively, 1% to 15% (or 2% to 10%) hydroxypropyl- β-cyclodextrin. Co-solvents include polysorbates (for example, polysorbate 20, 60, and 80), polyoxyethylene/polyoxypropylene surfactants (e.g., Pluronic^{®} F68, F84, F127, and P103), cyclodextrin, fatty-acid triglycerides, glycerol, polyethylene glycol, other solubility agents such as octoxynol 40 and tyloxapol, or other agents known to those skilled in the art and mixtures thereof. The amount of solubility enhancer used will depend on the amount of fluoroquinolone in the composition, with more solubility enhancer used for greater amounts of fluoroquinlones. Typically, solubility enhancers are employed at a level of from 0.01% to 20% (alternatively, 0.1% to 5%, or 0.1% to 2%) by weight depending on the ingredient.

The use of viscosity enhancing agents to provide the compositions of the invention with viscosities greater than the viscosity of simple aqueous solutions may be desirable to increase absorption of the active compounds by the target tissues or to increase the retention time therein. Such viscosity enhancing agents include, for example, polyvinyl alcohol, polyvinyl pyrrolidone, methyl cellulose, hydroxypropylmethyl cellulose, hydroxyethyl cellulose, carboxymethyl cellulose, hydroxypropyl cellulose or other agents known to those skilled in the art. Such agents are typically employed at a level of from 0.01% to 10% (alternatively, 0.1% to 5%, or 0.1% to 2%) by weight.

Suitable surfactants include polyvinyl pyrolidone, polyvinyl alcholol, polyethylene glycol, ethylene glycol, and propylene glycol. Other surfactants are polysorbates (such as polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of Tween^{®} 80, Tween^{®} 60, Tween^{®} 20), poloxamers (synthetic block polymers of ethylene oxide and propylene oxide, such as those commonly known by their trade names of Pluronic^{®}; e.g., Pluronic^{®} F127 or Pluronic^{®} F108)), or poloxamines (synthetic block polymers of ethylene oxide and propylene oxide attached to ethylene diamine, such as those commonly known by their trade names of Tetronic^{®}; e.g., Tetronic^{®} 1508 or Tetronic^{®} 908, etc., other nonionic surfactants such as Brij^{®}, Myrj^{®}, and long chain fatty alcohols (i.e., oleyl alcohol, stearyl alcohol, myristyl alcohol, docosohexanoyl alcohol, etc.) with carbon chains having about 12 or more carbon atoms (e.g., such as from about 12 to about 24 carbon atoms). The surfactant helps a topical formulation to spread on the surface of narrow passages.

In one aspect, it may be desirable to include in a composition of the present invention at least another anti-inflammatory agent. Preferred anti-inflammatory agents include the well-known non-steroidal anti-inflammatory drugs ("NSAIDs").

Non-limiting examples of the NSAIDs are: aminoarylcarboxylic acid derivatives (e.g., enfenamic acid, etofenamate, flufenamic acid, isonixin, meclofenamic acid, mefenamic acid, niflumic acid, talniflumate, terofenamate, tolfenamic acid), arylacetic acid derivatives (e.g., aceclofenac, acemetacin, alclofenac, amfenac, amtolmetin guacil, bromfenac, bufexamac, cinmetacin, clopirac, diclofenac sodium, etodolac, felbinac, fenclozic acid, fentiazac, glucametacin, ibufenac, indomethacin, isofezolac, isoxepac, lonazolac, metiazinic acid, mofezolac, oxametacine, pirazolac, proglumetacin, sulindac, tiaramide, tolmetin, tropesin, zomepirac), arylbutyric acid derivatives (e.g., bumadizon, butibufen, fenbufen, xenbucin), arylcarboxylic acids (e.g., clidanac, ketorolac, tinoridine), arylpropionic acid derivatives (e.g., alminoprofen, benoxaprofen, bermoprofen, bucloxic acid, carprofen, fenoprofen, flunoxaprofen, flurbiprofen, ibuprofen, ibuproxam, indoprofen, ketoprofen, loxoprofen, naproxen, oxaprozin, piketoprolen, pirprofen, pranoprofen, protizinic acid, suprofen, tiaprofenic acid, ximoprofen, zaltoprofen), pyrazoles (e.g., difenamizole, epirizole), pyrazolones (e.g., apazone, benzpiperylon, feprazone, mofebutazone, morazone, oxyphenbutazone, phenylbutazone, pipebuzone, propyphenazone, ramifenazone, suxibuzone, thiazolinobutazone), salicylic acid derivatives (e.g., acetaminosalol, aspirin, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide o-acetic acid, salicylsulfuric acid, salsalate, sulfasalazine), thiazinecarboxamides (e.g., ampiroxicam, droxicam, isoxicam, lornoxicam, piroxicam, tenoxicam), ε-acetamidocaproic acid, S-(5'-adenosyl)-L-methionine, 3-amino-4-hydroxybutyric acid, amixetrine, bendazac, benzydamine, α-bisabolol, bucolome, difenpiramide, ditazol, emorfazone, fepradinol, guaiazulene, nabumetone, nimesulide, oxaceprol, paranyline, perisoxal, proquazone, superoxide dismutase, tenidap, zileuton, their physiologically acceptable salts, combinations thereof, and mixtures thereof. In one embodiment, the NSAID is diclofenac, furbiprofen, or ketorolac.

Other non-steroidal anti-inflammatory agents include the cyclooxygenase type II selective inhibitors, such as celecoxib, and etodolac; PAF (platelet activating factor) antagonists, such as apafant, bepafant, minopafant, nupafant, and modipafant; PDE (phosphodiesterase) IV inhibitors, such as ariflo, torbafylline, rolipram, filaminast, piclamilast, cipamfylline, and roflumilast; inhibitors of cytokine production, such as inhibitors of the NF-κB transcription factor; or other anti-inflammatory agents known to those skilled in the art. In one embodiment, the non-steroidal anti-inflammatory agent is celecoxib.

The concentrations of each of the anti-inflammatory agents that may be included in the compositions of the present invention will vary based on the agent or agents selected and the type of inflammation being treated. The concentrations will be sufficient to reduce, treat, or prevent inflammation in the targeted tissues following application of a composition of the present invention to those tissues. Such concentrations are typically in the range from 0.0001 to 3% by weight (or alternatively, from 0.01 to 2%, or from 0.05% to 1%, or from 0.01% to 0.5%, by weight).

The following examples are provided to further illustrate compositions of the present invention,

### EXAMPLE 1: Solution

| Ingredient | Amount (% by weight) |
|---|---|
| Compound having Formula IV | 0.2 |
| Hydroxypropylmethylcellulose ("HPMC") | 0.5 |
| Benzakonium chloride ("BAK") | 0.01 |
| Pluronic® F127 | 0.1 |
| EDTA | 0.1 |
| NaCl | 0.25 |
| Phosphate buffer (0.05M, pH = 5.0) | q.s. to 100 |

An appropriate proportion (shown in the above table) of Pluronic^{®} F127 is added to phosphate buffer in a sterilized stainless steel jacketed vessel equipped with a stirring mechanism, at a temperature in the range from 50 to 60° C. The resulting buffer solution is heated to 61 to 75° C. At a temperature of about 66° C, an appropriate amount of BAK is added to the buffer solution while mixing three to ten minutes. At a temperature of 75° C, an appropriate amount of the compound having Formula IV is added to the contents of the vessel over a period of three to five minutes while mixing continues. EDTA and NaCl are then added to the mixture while mixing continues for five more minutes at 75° C. The resulting mixture is cooled to 25 to 30° C. The final composition is packaged in appropriate containers.

### EXAMPLE 2: Solution

A procedure similar to that of Example 1 is used to produce this solution.

| Ingredient | Amount (% by weight) |
|---|---|
| Compound having Formula IV | 0.35 |
| Mannitol | 4.5 |
| Benzakonium chloride ("BAK") | 0.005 |
| Polysorbate 80 | 0.1 |
| EDTA | 0.05 |
| Sodium acetate | 0.03 |
| Acetic acid | 0.04 |
| Purified water | q.s. to 100 |

### EXAMPLE 3: Solution

A procedure similar to that of Example I is used to produce this solution having the following composition.

| Ingredient | Amount (% by weight) |
|---|---|
| Compound having Formula IV | 0.2 |
| Dexamethasone | 0.1 |
| Hydroxypropylmethyl cellulose ("HPMC") | 0.5 |
| Alexidine | 0.01 |
| Brij® surfactant | 0.1 |
| EDTA | 0.1 |
| Citrate buffer (0.02M sodium citrate, pH = 5.0) | q.s. to 100 |

### EXAMPLE 4: Solution

A procedure similar to that of Example 1 is used to produce this solution having the following composition.

| Ingredient | Amount (% by weight) |
|---|---|
| Compound 8 of Table 1 | 0.3 |
| Colecoxib | 0.15 |
| Propylene glycol | 0.5 |
| Alexidine | 0.01 |
| Tyloxapol | 0.1 |
| EDTA | 0.1 |
| Citrate buffer (0.02M sodium citrate, pH = 5) | q.s. to 100 |

### EXAMPLE 5: Suspension

A procedure similar to that of Example 1 is used to produce this solution having the following composition.

| Ingredient | Amount (% by weight) |
|---|---|
| Compound having Formula IV | 0.3 |
| Triamcinolone, micronized USP | 0.2 |
| Hydroxyethyl cellulose | 0.25 |
| BAK | 0.01 |
| Tyloxapol | 0.05 |
| EDTA | 0.01 |
| NaCI | 0.3 |
| Na₂SO₄ | 1.2 |
| Sulfuric acid and/or NaOH | q.s. for pH adjustment to 5.5 |
| Citrate buffer (0.02M sodium citrate, pH = 5.0) | q.s. to 100 |

### EXAMPLE 6: Emulsion

A modification of the procedure of Example 1 is used to produce this emulsion having the composition shown in the table below.

Polysorbate 60 (Tween^{®} 60) is added to water in a first sterilized stainless steel jacketed vessel, equipped with a stirring mechanism, at a temperature of 50° C to 60° C in amounts corresponding the proportions shown in the table below. The resulting aqueous solution is heated to 61 ° C to 75° C. At a temperature of 66° C, benzyl alcohol (a preservative) is added to the aqueous solution while mixing three to ten minutes. At a temperature of 75° C, appropriate amounts of the compound having Formula IV and loteprednole etabonate are added to Mygliol oil in a second sterilized vessel, also equipped with a stirring mechanism, over a period of three to five minutes while stirring continues. Sorbitan monostearate and cetyl stearyl alcohol are added to the oil mixture. The resulting oil mixture is heated to a temperature in the range from 62° C to 75° C. The oil mixture is then added with vigorous mixing to the aqueous solution in the first vessel at a temperature of 66° C over a period of three to five minutes. Sodium sulfate and sulfuric acid and/or sodium hydroxide are added to the mixture to adjust pH to 5.5. The resulting composition is cooled to 35° C to 45° C and homogenized by mixing with a high shear emulsifier or running through a homogenizer. The composition is further cooled to 25° C to 30° C. The final composition is packaged in appropriate containers.

| Ingredient | Amount (% by weight) |
|---|---|
| Compound having Formula IV | 0.5 |
| Loteprednol etabonate | 0.2 |
| Polysorbate 60 | 1 |
| Sorbitan monostearate (an emulsifier) | 1.5 |
| Cetyl stearyl alcohol (an emulsion stabilizer) | 1.5 |
| Benzyl alcohol | 0.5 |
| Miglyol oil | 14.5 |
| Na₂SO₄ | 1.2 |
| Sulfuric acid and/or NaOH | q.s. for pH adjustment to 5.5 |
| Purified water | q.s. to 100 |

Typically, the oil used in an emulsion is a non-irritating emollient oil. Illustrative but non-limiting examples thereof include a mineral oil, vegetable oil, and a reformed vegetable oil of known composition. More specific but non-limiting examples of the oil can be selected from the group consisting of peanut oil, sesame seed oil, cottonseed oil, and a medium chain (C₆ to C₁₂) triglycerides (e.g., Miglyol Neutral Oils 810, 812, 818, 829, 840, etc., available from Huls America Inc.). Typical emulsifiers employed can be selected from the group consisting of sorbitan monostearate and polysorbate. Preferably, the emulsifiers are nonionic. The emulsifiers can be employed in an amount of 1.5 to 6.5% by weight of the composition, and preferably, 3 to 5% by weight of the composition. The hydrophobic phase of the emulsion can be in an amount of 15 to 25% by weight of the composition, and preferably, 18 to 22% by weight of the composition.

### EXAMPLE 7: Emulsion

A procedure similar to that of Example 6 is used to produce this emulsion having the following composition.

| Ingredient | Amount (% by weight) |
|---|---|
| Compound 13 of Table 1 | 0.5 |
| Triamcinolone, micronized USP | 0.2 |
| Polysorbate 60 | 1 |
| Sorbitan monostearate | 1.5 |
| Cetyl stearyl alcohol | 1.5 |
| Benzyl alcohol | 0.5 |
| Miglyol oil | 14.5 |
| Na₂SO₄ | 1.2 |
| Sulfuric acid and/or NaOH | q.s. for pH adjustment to 5.5 |
| Purified water | q.s. to 100 |

### EXAMPLE 8: Ointment

A procedure similar to that of Example 1 is used to produce this solution having the following composition.

| Ingredient | Amount (% by weight) |
|---|---|
| Compound having Formula IV | 0.3 |
| White petrolatum USP | 50 |
| Propylene glycol | 5 |
| Glycerin | 5 |
| Tween^{®} 20 | 2 |
| Vitamin E | 1 |
| BAK | 0.1 |
| Mineral oil | q.s. to 100 |

### EXAMPLE 9: Ointment

A procedure similar to that of Example 1 is used to produce this solution having the following composition.

| Ingredient | Amount (% by weight) |
|---|---|
| Compound having Formula VI | 0.3 |
| Dexamethasone | 0.15 |
| White petrolatum USP | 50 |
| Propylene glycol | 5 |
| Glycerin | 5 |
| Tween^{®} 20 | 2 |
| Vitamin E | 1 |
| Vitamin D | 0.5 |
| BAK | 0.1 |
| Mineral oil | q.s. to 100 |

### EXAMPLE 10: Tablet

The ingredients shown in the table below are blended together in a blender, such as a ribbon blender. Other types of blenders that are well known to people skilled in the art of powder mixing also can be used. The mixture is fed through a tableting press at condition suitable for producing pharmaceutical tablets.

| Ingredient | Amount (% by weight) |
|---|---|
| Compound having Formula IV | 0.3 |
| Microcrystalline cellulose | 20 |
| Magnesium stearate | 2 |
| Mannitol | 65 |
| Starch | q.s. to 100 |

### COMPARISON OF SIDE EFFECTS OF GLUCOCORTICOIDS AND PRESENT FLUOROQUINOLONES

One of the most frequent undesirable actions of a glucocorticoid therapy is steroid diabetes. The reason for this undesirable condition is the stimulation of gluconeogenesis in the liver by the induction of the transcription of hepatic enzymes involved in gluconeogenesis and metabolism of free amino acids that are produced from the degradation of proteins (catabolic action of glucocorticoids). A key enzyme of the catabolic metabolism in the liver is the tyrosine aminotransferase ("TAT"). The activity of this enzyme can be determined photometrically from cell cultures of treated rat hepatoma cells. Thus, the gluconeogenesis by a glucocorticoid can be compared to that of a fluroquinolone disclosed herein by measuring the activity of this enzyme. For example, in one procedure, the cells are treated for 24 hours with the test substance (a fluroquinolone or glucocorticoid), and then the TAT activity is measured. The TAT activities for the selected fluroquinolone and glucocorticoid are then compared. Other hepatic enzymes can be used in place of TAT, such as phosphoenolpyruvate carboxykinase, glucose-6-phosphatase, or fructose-2,6-biphosphatase. Alternatively, the levels of blood glucose in an animal model may be measured directly and compared for individual subjects that are treated with a glucocorticoid for a selected condition and those that are treated with a fluroquinolone for the same condition.

Another undesirable result of glucocorticoid therapy is GC-induced-cataract. The cataractogenic potential of a compound or composition may be determined by quantifying the effect of the compound or composition on the flux of potassium ions through the membrane of lens cells (such as mammalian lens epithelial cells) in vitro. Such an ion flux may be determined by, for example, electrophysiological techniques or ion-flux imaging techniques (such as with the use of fluorescent dyes). An exemplary in-vitro method for determining the cataractogenic potential of a compound or composition is disclosed in U.S. Patent Application Publication 2004/0219512.

Still another undesirable result of glucocorticoid therapy is hypertension. Blood pressure of similarly matched subjects treated with glucocorticoid and a fluroquinolone of the present invention for an inflammatory condition may be measured directly and compared.

Yet another undesirable result of glucocorticoid therapy is increased intraocular pressure ("IOP") in the subject. IOP of similarly matched subjects treated with glucocorticoid and a fluroquinolone of the present invention for a condition may be measured directly and compared.

## Claims

1. A composition comprising a fluoroquinolone having Formula I, II, III, IV, V, VI, VII, or VIII, or a salt thereof for use in a medicament for modulating inflammatory endophthalmitis in a subject, wherein said fluoroquinolone is present in an effective amount for said modulating 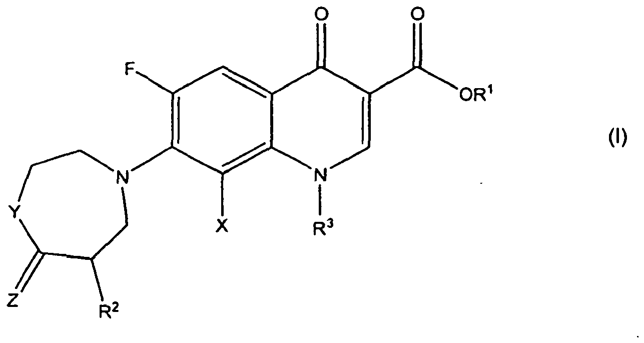 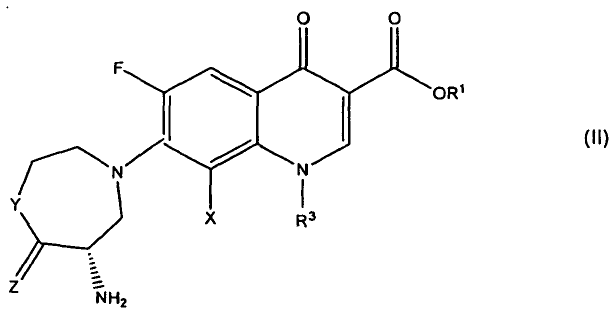 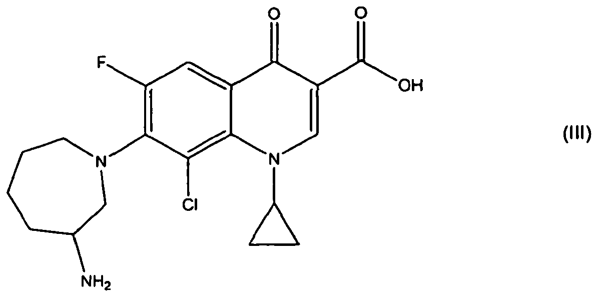 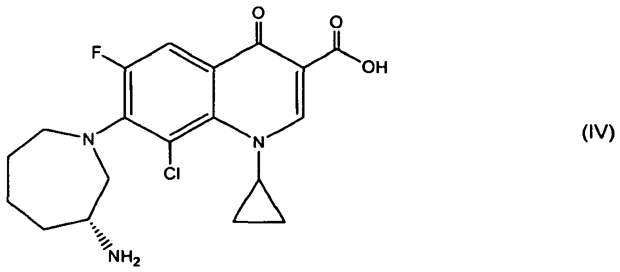 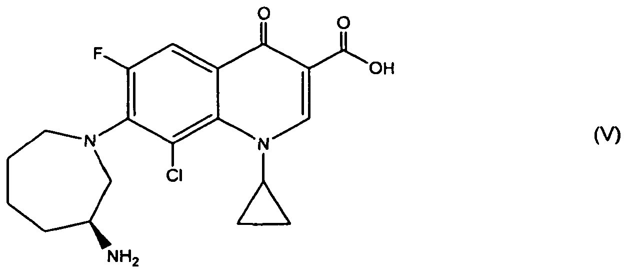 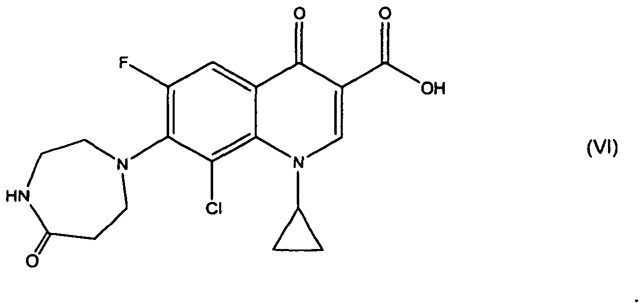 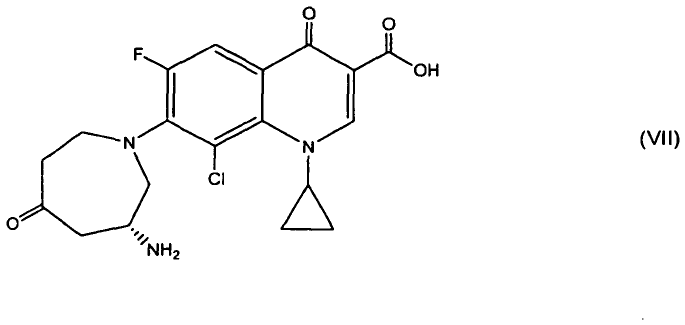 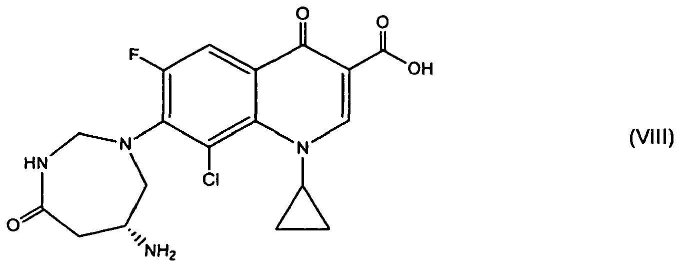
wherein R¹ is selected from the group consisting of hydrogen, unsubstituted lower alkyl groups, substituted lower alkyl groups, cycloalkyl groups, unsubstituted C₅-C₂₄ aryl groups, substituted C₅-C₂₄ aryl groups, unsubstituted C₅-C₂₄ heteroaryl groups, substituted C₅-C₂₄ heteroaryl groups, and groups that can be hydrolyzed in living bodies;
R² is selected from the group consisting of hydrogen, unsubstituted amino group, and amino groups substituted with one or two lower alkyl groups;
R³ is selected from the group consisting of hydrogen, unsubstituted lower alkyl groups, substituted lower alkyl groups, cycloalkyl groups, unsubstituted lower alkoxy groups, substituted lower alkoxy groups, unsubstituted C₂-C₂₄ aryl groups, substituted C₅-C₂₄ aryl groups, unsubstituted C₅-C₂₄ heteroaryl groups, substituted C₅-C₂₄ heteroaryl groups, unsubstituted C₅-C₂₄ aryloxy groups, substituted C₅-C₂₄ aryloxy groups, unsubstituted C₅-C₂₄ heteroaryloxy groups, substituted C₅-C₂₄ heteroaryloxy groups, and groups that can be hydrolyzed in living bodies;
X is selected from the group consisting of halogen atoms;
Y is selected from the group consisting of CH₂, O, S, SO, SO₂, and NR⁴, wherein R⁴ is selected from the group consisting of hydrogen, unsubstituted lower alkyl groups, substituted lower alkyl groups, and cycloalkyl groups; and
Z is selected from the group consisting of oxygen and two hydrogen atoms.

2. The composition of claim 1, wherein said endophthalmitis is selected from the group consisting of post-operative endophthalmitis, post-traumatic endophthalmitis, non-infectious endophthalmitis, panophthalmitis, hematogenous endophthalmitis, and combinations thereof.

3. The composition of claim 1, wherein said endophthalmitis comprises a result of an infection.

4. The composition of claim 1, wherein R¹ is selected from the group consisting of hydrogen, C₁-C₅ substituted and unsubstituted alkyl groups, C₃-C₁₀ cycloalkyl groups, C₅-C₁₄ substituted and unsubstituted aryl groups, C₅-C₁₄ substituted and unsubstituted heteroaryl groups, and groups that can be hydrolyzed in living bodies; R² is selected from the group consisting of unsubstituted amino group and amino groups substituted with one or two C₁-C₅ alkyl groups; and R³ is selected from the group consisting of hydrogen, C₁-C₅ substituted and unsubstituted alkyl groups, C₃-C₁₀ cycloalkyl groups, C₁-C₅ substituted and unsubstituted alkoxy groups, C₅-C₁₄ substituted and unsubstituted aryl groups, C₅-C₁₄ substituted and unsubstituted heteroaryl groups, and C₅-C₁₄ substituted and unsubstituted aryloxy groups.

5. The composition of claim 1, wherein R³ is selected from the group consisting of C₃-C₁₀ cycloalkyl groups.

6. The composition of claim 1, wherein X is Cl.

7. The composition of claim 6, wherein Y is CH₂.

8. The composition of claim 6, wherein Z comprises two hydrogen atoms.

9. The composition of claim 1, wherein Y is NH, Z is O, and X is Cl.

10. The composition of claim 1, wherein the medicament comprises a solution, emulsion, dispersion, suspension, ointment, or gel.

11. The composition of claim 1, wherein the fluoroquinolone or salt thereof is present in an amount from about 0.0001% to 10% by weight of the composition.

12. The composition of claim 11, wherein the composition further comprises a non-steroidal anti-inflammatory drug.

## Patentansprüche

1. Eine Zusammensetzung umfassend ein Fluoroquinolon, welches die Formel I, II, III, IV, V, VI, VII oder VIII oder ein Salz davon aufweist, zur Verwendung in einem Medikament für die Modulierung entzündlicher Endophthalmitis in einem Subjekt, wobei besagtes Fluoroquinolon in einer für besagte Modulierung wirksamen Menge vorhanden ist 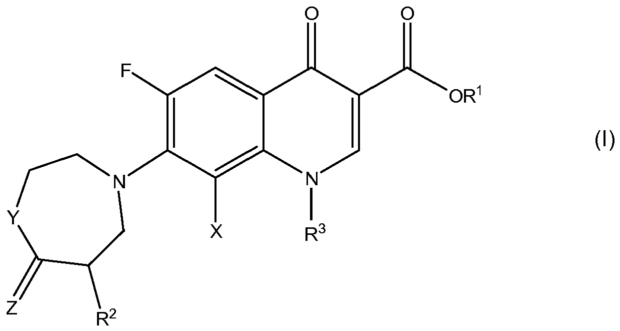 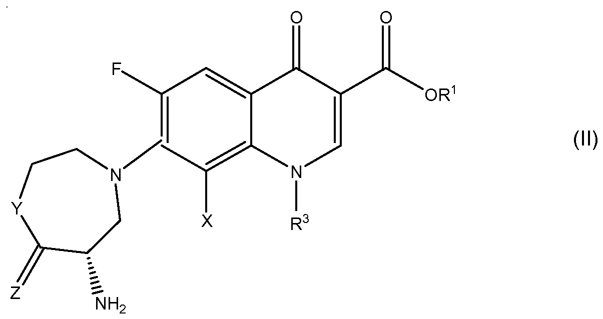 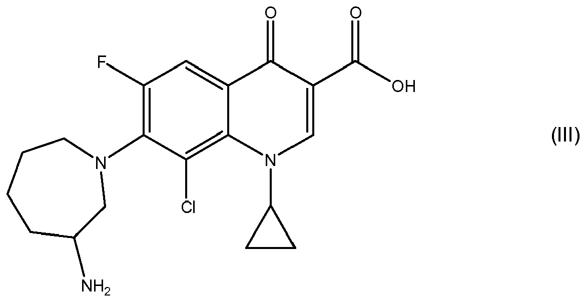 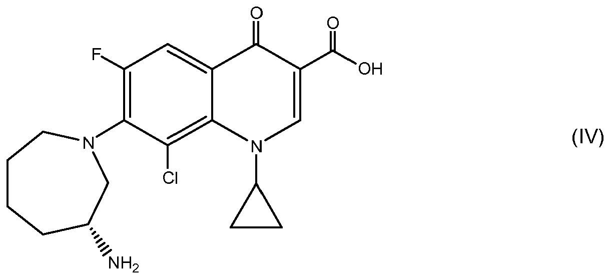 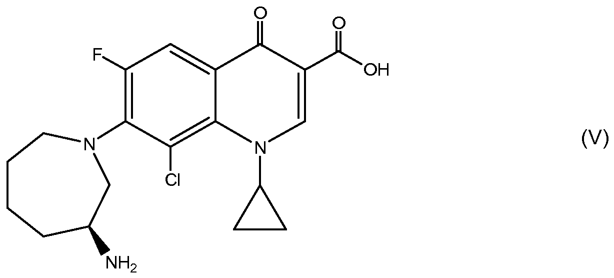 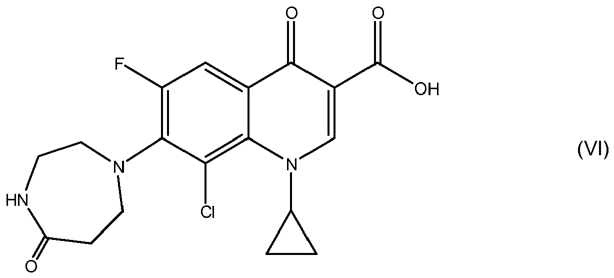 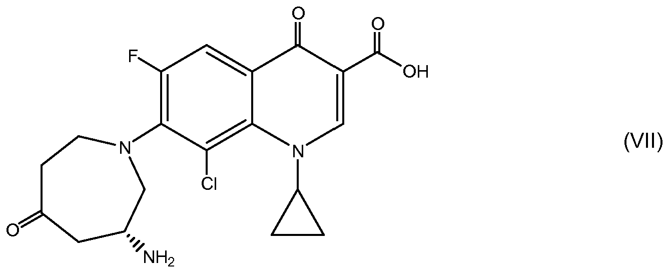 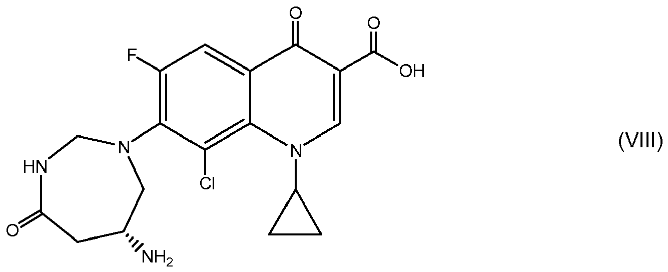
wobei R¹ ausgewählt ist aus der Gruppe bestehend aus Wasserstoff, unsubstituierten Niederalkylgruppen, substituierten Niederalkylgruppen, Cycloalkylgruppen, unsubstituierten C₅-C₂₄ Arylgruppen, substituierten C₅-C₂₄ Arylgruppen, unsubstituierten C₅-C₂₄ Heteroarylgruppen, substituierten C₅-C₂₄ Heteroarylgruppen und Gruppen, welche in lebenden Körpern hydrolisiert werden können;
R² ausgewählt ist aus der Gruppe bestehend aus Wasserstoff, unsubstituierten Aminogruppen und Aminogruppen, die mit einer oder zwei Niederalkylgruppen substituiert sind;
R³ ausgewählt ist aus der Gruppe bestehend aus Wasserstoff, unsubstituierten Niederalkylgruppen, substituierten Niederalkylgruppen, Cycloalkylgruppen, unsubstituierten Niederalkoxygruppen, substituierten Niederalkoxygruppen, unsubstituierten C₅-C₂₄ Arylgruppen, substituierten C₅-C₂₄ Arylgruppen, unsubstituierten C₅-C₂₄ Heteroarylgruppen, substituierte C₅-C₂₄ Heteroarylgruppen, unsubstituierte C₅-C₂₄ Aryloxygruppen, substituierte C₅-C₂₄ Aryloxygruppen, unsubstituierte C₅-C₂₄ Heteroaryloxygruppen, substituierte C₅-C₂₄ Heteroaryloxygruppen und Gruppen, welche in lebenden Körpern hydrolisiert werden können;
X ausgewählt ist aus der Gruppe bestehend aus Halogenatomen;
Y ausgewählt ist aus der Gruppe bestehend aus CH₂, O, S, SO, SO₂ und NR⁴, wobei R⁴ ausgewählt ist aus der Gruppe bestehend aus Wasserstoff, unsubstituierten Niederalkylgruppen, substituierten Niederalkylgruppen und Cycloalkylgruppen und
Z ausgewählt ist aus der Gruppe bestehend aus Sauerstoff und zwei Wasserstoffatomen.

2. Die Zusammensetzung gemäß Anspruch 1, wobei besagte Endophthalmitis ausgewählt ist aus der Gruppe bestehend aus postoperativer Endophthalmitis, posttraumatischer Endophthalmitis, nicht-infektiöser Endophthalmitis, Panophthalmitis, hämatogener Endophthalmitis und Kombinationen davon.

3. Die Zusammensetzung gemäß Anspruch 1, wobei besagte Endophthalmitis das Resultat einer Infektion umfasst.

4. Die Zusammensetzung gemäß Anspruch 1, wobei R¹ ausgewählt ist aus der Gruppe bestehend aus Wasserstoff, C₁-C₅ substituierten und unsubstituierten Alkylgruppen, C₃-C₁₀ Cycloalkylgruppen, C₅-C₁₄ substituierten und unsubstituierten Arylgruppen, C₅-C₁₄ substituierten und unsubstituierten Heteroarylgruppen und Gruppen, welche in lebenden Körpern hydrolisiert werden können; R² ausgewählt ist aus der Gruppe bestehend aus unsubstituierten Aminogruppen und Aminogruppen, die mit einer oder zwei C₁-C₅ Alkylgruppen substituiert sind und R³ ausgewählt ist aus der Gruppe bestehend aus Wasserstoff, C₁-C₅ substituierten und unsubstituierten Alkylgruppen, C₃-C₁₀ Cycloalkylgruppen, C₁-C₅ substituierten und unsubstituierten Alkoxygruppen, C₅-C₁₄ substituierten und unsubstituierten Arylgruppen, C₅-C₁₄ substituierten und unsubstituierten Heteroarylgruppen und C₅-C₁₄ substituierten und unsubstituierten Aryloxygruppen.

5. Die Zusammensetzung gemäß Anspruch 1, wobei R³ ausgewählt ist aus der Gruppe bestehend aus C₃-C₁₀ Cycloalkylgruppen.

6. Die Zusammensetzung gemäß Anspruch 1, wobei X Cl ist.

7. Die Zusammensetzung gemäß Anspruch 6, wobei Y CH₂ ist.

8. Die Zusammensetzung gemäß Anspruch 6, wobei Z zwei Halogenatome umfasst.

9. Die Zusammensetzung gemäß Anspruch 1, wobei Y NH ist, Z O ist und X Cl ist.

10. Die Zusammensetzung gemäß Anspruch 1, wobei das Medikament eine Lösung, eine Emulsion, eine Dispersion, eine Suspension, eine Salbe oder ein Gel umfasst.

11. Die Zusammensetzung gemäß Anspruch 1, wobei das Fluoroquinolon oder Salz davon in einer Menge von etwa 0,0001 % bis 10 % bezogen auf das Gewicht der Zusammensetzung vorhanden ist.

12. Die Zusammensetzung gemäß Anspruch 11, wobei die Zusammensetzung weiterhin einen nicht-steroidalen entzündungshemmenden Wirkstoff umfasst.

## Revendications

1. Composition comprenant une fluoroquinolone de formule I, II, III, IV, V, VI, VII ou VIII, ou un sel de celle-ci, pour utilisation dans un médicament destiné à moduler une endophtalmie inflammatoire chez un sujet, dans laquelle ladite fluoroquinolone est présente en une quantité efficace pour ladite modulation : 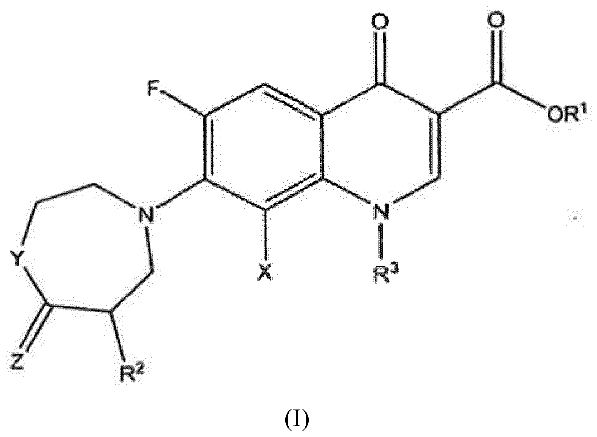 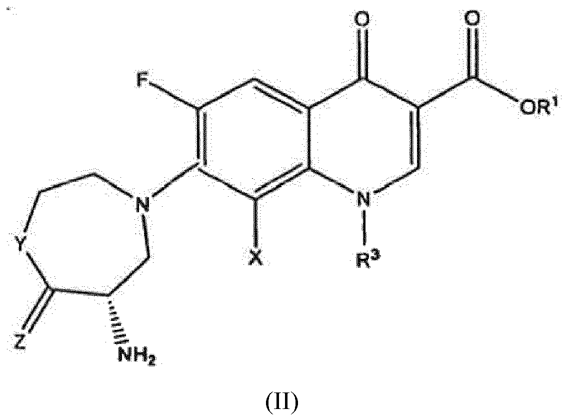 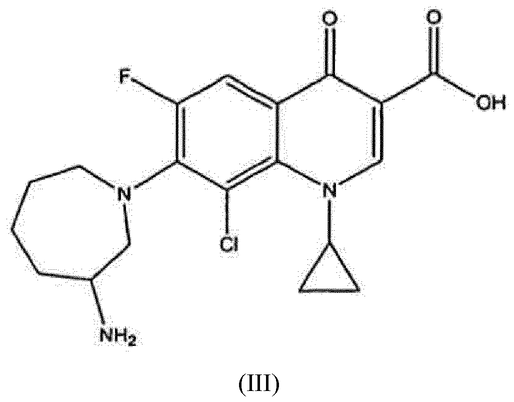 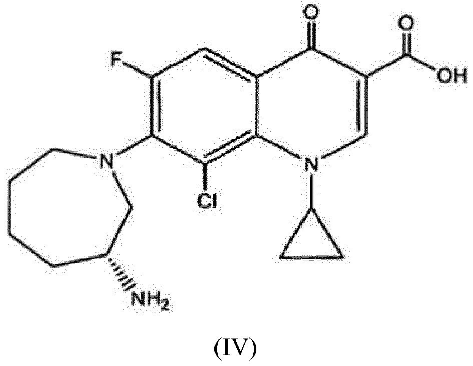 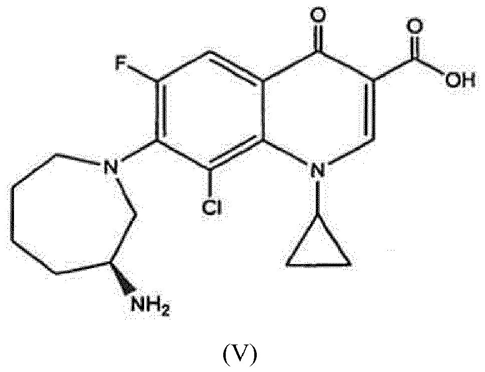 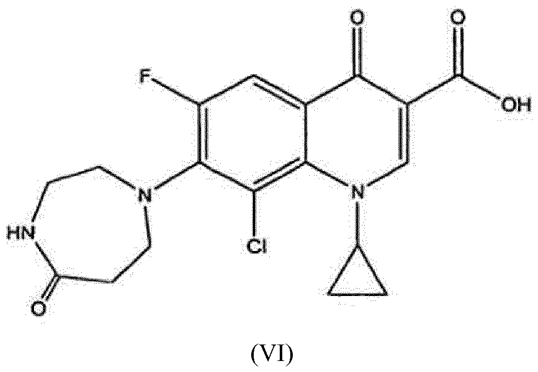 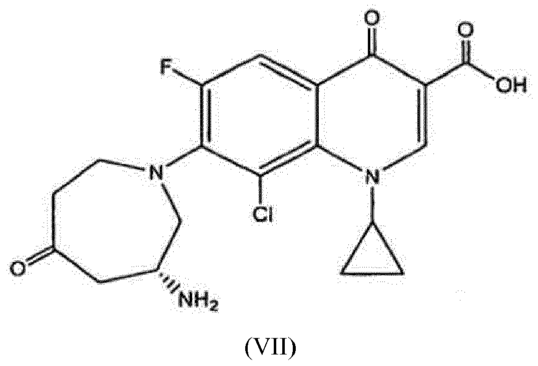 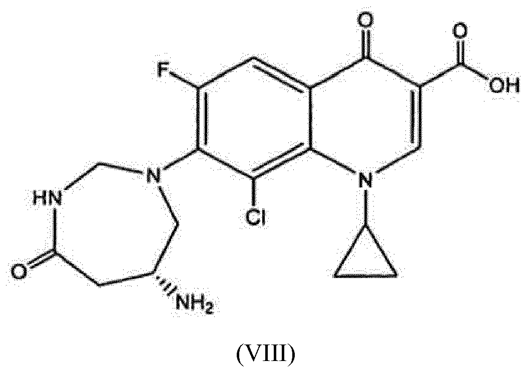
où R¹ est choisi dans l'ensemble constitué par l'hydrogène, les groupes alkyle inférieur non substitués, les groupes alkyle inférieur substitués, les groupes cycloalkyle, les groupes aryle en C₅ à C₂₄ non substitués, les groupes aryle en C₅ à C₂₄ substitués, les groupes hétéroaryle en C₅ à C₂₄ non substitués, les groupes hétéroaryle en C₅ à C₂₄ substitués, et les groupes qui peuvent être hydrolysés dans les corps vivants ;
R² est choisi dans l'ensemble constitué par l'hydrogène, le groupe amino non substitué, et les groupes amino substitués par un ou deux groupes alkyle inférieur ; R³ est choisi dans l'ensemble constitué par l'hydrogène, les groupes alkyle inférieur non substitués, les groupes alkyle inférieur substitués, les groupes cycloalkyle, les groupes alcoxy inférieur non substitués, les groupes alcoxy inférieur substitués, les groupes aryle en C₅ à C₂₄ non substitués, les groupes aryle en C₅ à C₂₄ substitués, les groupes hétéroaryle en C₅ à C₂₄ non substitués, les groupes hétéroaryle en C₅ à C₂₄ substitués, les groupes aryloxy en C₅ à C₂₄ non substitués, les groupes aryloxy en C₅ à C₂₄ substitués, les groupes hétéroaryloxy en C₅ à C₂₄ non substitués, les groupes hétéroaryloxy en C₅ à C₂₄ substitués, et les groupes qui peuvent être hydrolysés dans les corps vivants ;
X est choisi dans l'ensemble constitué par les atomes d'halogène ;
Y est choisi dans l'ensemble constitué par CH₂, O, S, SO, SO₂ et NR⁴ où R⁴ est choisi dans l'ensemble constitué par l'hydrogène, les groupes alkyle inférieur non substitués, les groupes alkyle inférieur substitués, et les groupes cycloalkyle ; et
Z est choisi dans l'ensemble constitué par l'oxygène et deux atomes d'hydrogène.

2. Composition selon la revendication 1, dans laquelle ladite endophtalmie est choisie dans l'ensemble constitué par une endophtalmie postopératoire, une endophtalmie post-traumatique, une endophtalmie non infectieuse, une panophtalmie, une endophtalmie hématogène, et leurs combinaisons.

3. Composition selon la revendication 1, dans laquelle ladite endophtalmie est le résultat d'une infection.

4. Composition selon la revendication 1, dans laquelle R¹ est choisi dans l'ensemble constitué par l'hydrogène, les groupes alkyle en C₁ à C₅ substitués et non substitués, les groupes cycloalkyle en C₃ à C₁₀, les groupes aryle en C₅ à C₁₄ substitués et non substitués, les groupes hétéroaryle en C₅ à C₁₄ substitués et non substitués, et les groupes qui peuvent être hydrolysés dans les corps vivants ; R² est choisi dans l'ensemble constitué par le groupe amino non substitué et les groupes amino substitués par un ou deux groupes alkyle en C₁ à C₅ et R³ est choisi dans l'ensemble constitué par l'hydrogène, les groupes alkyle en C₁ à C₅ substitués et non substitués, les groupes cycloalkyle en C₃ à C₁₀, les groupes alcoxy en C₁ à C₅ substitués et non substitués, les groupes aryle en C₅ à C₁₄ substitués et non substitués, les groupes hétéroaryle en C₅ à C₁₄ substitués et non substitués, et les groupes aryloxy en C₅ à C₁₄ substitués et non substitués.

5. Composition selon la revendication 1, dans laquelle R³ est choisi dans l'ensemble constitué par les groupes cycloalkyle en C₃ à C₁₀.

6. Composition selon la revendication 1, dans laquelle X est Cl.

7. Composition selon la revendication 6, dans laquelle Y est CH₂.

8. Composition selon la revendication 6, dans laquelle Z comprend deux atomes d'hydrogène.

9. Composition selon la revendication 1, dans laquelle Y est NH, Z est O, et X est Cl.

10. Composition selon la revendication 1, dans laquelle le médicament comprend une solution, une émulsion, une dispersion, une suspension, une pommade, ou un gel.

11. Composition selon la revendication 1, dans laquelle la fluoroquinolone ou son sel est présent en une quantité d'environ 0,0001 % à 10 % en poids de la composition.

12. Composition selon la revendication 11, laquelle composition comprend en outre un médicament anti-inflammatoire non stéroïdien.
